# EUROPEAN PATENT APPLICATION

(11) **EP 2 193 807 A1**
(43) Date of publication of application: **09.06.2010**
(21) Application number: 08828474.0
(22) Date of filing: 22.08.2008
(51) Int. Cl.: A61K 45/00, C12N 5/10, C12N 15/09

(54) **CYCLOSPORIN A-BINDING PROTEIN**

(30) Priority: 24.08.2007 JP 2007217755
(71) Applicant: Sapporo Medical University, Hokkaido 060-0061 (JP); Tokyo University of Science, Tokyo 162-8601 (JP); Kyoto University, Sakyo-ku Kyoto 606-8501 (JP); Sapporo Immuno Diagnostic Laboratory, Hokkaido 001-0922 (JP)
(72) Inventor: SAHARA, Hiroeki, Sapporo-shi Hokkaido 060-8556 (JP); MORI, Yoko, Sapporo-shi Hokkaido 060-8556 (JP); TAKAHASHI, Nobuaki, Sapporo-shi Hokkaido 060-8556 (JP); SATO, Noriyuki, Sapporo-shi Hokkaido 060-8556 (JP); SUGAWARA, Fumio, Noda-shi Chiba 278-8510 (JP); SAKAGUCHI, Kengo, Noda-shi Chiba 278-8510 (JP); MOROHASHI, Kengo, Columbus, Ohio 43210 (US); IWABATA, Kazuki, Noda-shi Chiba 278-8510 (JP); WATASHI, Koichi, Kyoto-shi Kyoto 606-8507 (JP); SHIMOTOHNO, Kunitada, Kyoto-shi Kyoto 606-8507 (JP); KIKUCHI, Kokichi, Sapporo-shi Hokkaido 001-0922 (JP); TSUTAE, Wataru, Sapporo-shi Hokkaido 001-0922 (JP); MIYASHITA, Hiroki, Sapporo-shi Hokkaido 001-0922 (JP)
(74) Representative: Müller-Wolff, Thomas
(86) International application number: PCT/JP2008/065015
(87) International publication number: WO 2009/028418

(57) **Abstract**

Abstract: Disclosed are: a composition and a method for the diagnosis and/or the treatment of a CSABP-related disease, the regulation of the proliferation of an RNA virus and the regulation of an RNA-metabolizing system, which targets a cyclosporine A-binding protein (CSABP); a method for the screening of a component that targets a CSABP; a composition and a method for the detection/inhibition of CsA, NS5B or cyclosphilin B by utilizing a CSABP; a promoter for a CSABP; a method for the screening of a substance capable of regulating the expression of a CSABP by utilizing the promoter; and a method for the determination of the occurrence or clinical stage of a CSABP-related disease.

## Description

### Technical Field

The present invention relates to a composition and a method, targeting a cyclosporin A (CsA)-binding protein (CSABP: Cyclosporin A binding protein), for the diagnosis and/or treatment of a CSABP-related disease, control of growth of an RNA virus, and control of an RNA-metabolizing system, a method for screening a component that targets a CSABP, and a composition and a method, utilizing a CSABP, for the detection/inhibition of CsA, NS5B, or cyclophilin B (CyPB). Furthermore, the present invention relates to a CSABP promoter, a method, utilizing the promoter, for screening a substance that controls expression of a CSABP, and a method for determining the onset or disease stage of a CSABP-related disease by detecting methylation of a CSABP gene.

### Background Art

It is known that persistent hepatitis C virus (HCV) infection in the liver is a main cause for chronic liver diseases such as chronic hepatitis, hepatic cirrhosis, and hepatocellular carcinoma. The onset of these chronic liver diseases in HCV-infected patients, who are estimated to number about 1.5 million in Japan, is currently a serious public health problem, and the development of an antiviral therapy for excluding HCV is an urgently required task.
With regard to current treatments for hepatitis C virus, administration of interferon (IFN) alone or a combination therapy of IFN and ribavirin are mainly employed. However, in reality the response rates to these treatments is 30% to 60% on average, and development of an anti-HCV therapy that can replace them has been desired. Under such circumstances, in addition to attempts to optimize the current mainly interferon (IFN)-based treatment methods, searching for new anti-HCV drugs has so far been carried out by many researchers and pharmaceutical companies.

For example, it is known that NS3, which is a viral protease, and NS5B, which is an RNA-dependant RNA polymerase, are enzymes essential for the growth of HCV; as an NS3 inhibitor BILN2061, etc. have been developed, and as an NS5B inhibitor JTK-003, a cycloalkyl heterocyclic compound described in Patent Document 1, etc. have been developed. Furthermore, ribozymes (Heptazyme, etc.), antisense oligodeoxynucleotides (ISIS-14803, etc.), siRNAs, etc. targeting HCV genome RNA have also been developed. Moreover, by screening using an HCV subgenomic replicon system (Non-Patent Document 1), which has recently been developed, various types of compounds having the ability to suppress HCV replication have been identified. With regard to such compounds, for example, there have been reported cytokines such as IFNγ (Non-Patent Document 2), IL-1 (Non-Patent Document 3), and TGFβ (Non-Patent Document 4), arsenic trioxide (Non-Patent Document 5), microtubule polymerization inhibitors such as vinblastine (Non-Patent Document 6), geranylgeranylated inhibitors such as lovastatin (Non-Patent Document 7), and CsA (Non-Patent Document 8, Non-Patent Document 9) and its derivatives (e.g. a modified cyclosporin described in Patent Document 2, a drug for the treatment of hepatitis C described in Patent Document 3) as a low-molecular-weight compound. However, there is not yet a therapeutic agent that is satisfactory in terms of therapeutic effect and safety, and further research effort is needed.

Patent Document 1: JP-A-2007-505114
Patent Document 2: JP-A-2007-504260
Patent Document 3: JP-A-2007-15926
Non-Patent Document 1: Lohmann V, Science. 1999; 285 (5424): 110-3
Non-Patent Document 2: Frese M et al., Hepatology. 2002; 35 (3): 694-703
Non-Patent Document 3: Zhu H and Liu C, J Virol. 2003; 77 (9): 5493-8
Non-Patent Document 4: Murata T et al., Virology. 2005; 331 (2): 407-17
Non-Patent Document 5: Hwang DR et al., Antimicrob Agents Chemother. 2004; 48 (8): 2876-82
Non-Patent Document 6: Bost AG et al., J Virol. 2003; 77 (7): 4401-8
Non-Patent Document 7: Ye J et al., Proc Natl Acad Sci U S A. 2003; 100 (26): 15865-70
Non-Patent Document 8: Watashi K et al., Hepatology. 2003; 38 (5): 1282-8
Non-Patent Document 9: Nakagawa M et al., Biochem Biophys Res Commun. 2004; 313 (1): 42-7
Non-Patent Document 10: Watashi K et al., Mol Cell. 2005; 19 (1): 111-22

### Disclosure of Invention

### Problems to be Solved by the Invention

It is an object of the present invention to provide a novel HCV therapeutic agent having an excellent therapeutic effect.

### Means for Solving the Problems

While carrying out an intensive investigation in order to achieve the above-mentioned object, the present inventors have discovered a protein (CSABP) that binds to CsA. During further research focusing on this CSABP, it has been found that said protein forms a trimer with CyPB and NS5B, which are necessary for the replication of HCV; the replication of HCV is suppressed by suppression of the expression of said protein and replication is promoted by enhancement of its expression and, moreover, CSABP is expressed in a plurality of tumor cells but there is hardly any expression in normal cells. Furthermore, in accordance with further research, a promoter region of CSABP and a transcription factor that is involved in the transcription of CSABP have been identified, and in addition it has been found that the promoter region can be methylated at high frequency, expression of CSABP can be enhanced by administration of an anti-methylating agent, and expression of CSABP can be induced in normal cells by administration of an inflammatory cytokine, and the present invention has thus been accomplished.
Although it has been suggested that CsA binds to CyPB and inhibits interaction between CyPB and NS5B, which is necessary for the replication of HCV, thus suppressing growth of HCV (Non-Patent Document 10), there have not been any reports that another protein binds to a dimer of CyPB and NS5B to thus form a trimer and that this protein is essential for the replication of HCV, and even a person skilled in the art could not anticipate the presence of such a protein.

That is, the present invention relates to the following:
(1) A composition for treating a CSABP-related disease, the composition containing a component that controls the abundance and/or function of a CSABP.
(2) The composition according to (1) above, wherein the component that controls the abundance and/or function of a CSABP is selected from the group consisting of a CSABP, a functional variant of a CSABP, a dominant negative variant of a CSABP, NS5B, CyPB, and CSABP-binding variants thereof, an anti-CSABP antibody, nucleic acids coding therefor, vectors expressing these, and a substance that controls CSABP gene expression.
(3) A composition for diagnosing a CSABP-related disease, the composition containing a component that detects a CSABP or a nucleic acid coding therefor.

(4) The composition according to (3) above, wherein the component that detects a CSABP or a nucleic acid coding therefor is selected from the group consisting of an anti-CSABP antibody, CsA and a derivative thereof, NS5B, CyPB, and CSABP-binding variants thereof, and a nucleic acid that specifically hybridizes with a CSABP-coding nucleic acid or a unique fragment thereof.
(5) The composition according to (1) to (4) above, wherein the CSABP-related disease is selected from the group consisting of an RNA virus infection, hepatitis C, an inflammatory disease, a state in which replication of an RNA virus is promoted, and a tumor.
(6) A method for screening a component that detects and/or controls a CSABP, the method including a step of making a test substance coexist with a CSABP or a fragment thereof.
(7) A composition for controlling the growth of an RNA virus, the composition containing a component that controls the abundance and/or function of a CSABP.
(8) A composition for detecting and/or inhibiting CsA and a derivative thereof, NS5B, or CyPB, the composition containing a CSABP or a functional variant thereof.

(9) A method for screening a substance that controls CSABP gene expression, the method including 1) a step of making a cell having a nucleic acid molecule containing a CSABP promoter and a reporter sequence operably linked thereto coexist with a test substance, and 2) a step of detecting an expression product of the reporter sequence.
(10) A method for screening a substance that controls CSABP gene expression, the method including a step of recovering a substance that binds to a CSABP promoter.
(11) A CSABP promoter, selected from the group consisting of i) a nucleic acid molecule that contains a base sequence represented by SEQ ID No: 37 or a part thereof, ii) a nucleic acid molecule that has one or more mutations in the base sequence of nucleic acid molecule i) but that still has equivalent functions to said nucleic acid molecule, iii) a nucleic acid molecule that hybridizes with a complementary strand of nucleic acid molecule i) or ii) or a fragment thereof under stringent conditions and has equivalent functions to said nucleic acid molecule, and iv) a nucleic acid molecule that has at least 70% homology to nucleic acid molecule i) or ii) and has equivalent functions to said nucleic acid molecule.
(12) A vector having a CSABP promoter.
(13) A cell having a nucleic acid molecule containing a CSABP promoter and a reporter sequence operably linked thereto.
(14) A method for determining the onset or disease stage of a CSABP-related disease, the method including a step of detecting methylation in a CSABP gene.

### Effects of the Invention

The present invention provides a novel therapeutic agent and diagnostic agent for a CSABP-related disease, including an RNA virus infection or a tumor, and in particular HCV infection, and treatment/diagnosis methods therefor, and it can be anticipated that it will greatly contribute to human medical care and veterinary care. In particular, since a CSABP is a host-derived protein, unlike a virus-derived protein the frequency of mutations is low, and the therapeutic agent of the present invention targeting a CSABP has the advantage that it is difficult for virus resistance to occur. Moreover, since the therapeutic agent of the present invention can turn cells in which a virus easily replicates due to the effect of an inflammatory cytokine back into a normal state in which it is difficult for a virus to replicate, it is possible to specifically suppress the growth of a virus without suppressing the inflammation itself, which is necessary for phylaxis.
Furthermore, in accordance with the screening method of the present invention, it becomes possible to search for a therapeutic agent having a novel mechanism of action.
Moreover, the present invention provides a new target substance in the control of growth of an RNA virus or an RNA metabolism system, thus increasing the choices for drugs and methods that can be utilized in these applications and thereby contributing to progress in life sciences, including human medicine and veterinary medicine.
Furthermore, in accordance with the determination method of the present invention, since it is possible to elucidate the onset or condition of a tumor, RNA virus infection, etc. from a new direction, it becomes possible to understand the condition of a disease more precisely.

### Brief Description of Drawings

[FIG. 1] A diagram showing the results of analysis by a northern blot method of expression of CSABP in various samples.
[FIG. 2] A diagram showing the results of analysis by an RT-PCR method of expression of CSABP in normal tissue.
[FIG. 3] A diagram showing the results of analysis by an RT-PCR method of expression of CSABP in various cancer cells.
[FIG. 4] A diagram showing the results of analysis by SDS-PAGE of molecular weight of recombinant CSABP protein (CSABP-FL).
[FIG. 5] A graph showing the results of a molecular association test between CsA and CSABP (CSABP-FL). The abscissa denotes CsA concentration (µM) and the ordinate denotes RU value.
[FIG. 6] A graph showing the results of measurement of CSABP (CSABP-C) ATPase activity in the presence or absence of RNA. The abscissa denotes reaction time (min) and the ordinate denotes radioactivity (cpm) of free ³²P. Furthermore, RNA(+) and RNA(-) denote the results of measurement in the presence and absence of RNA respectively.

[FIG. 7] A graph evaluating the effect of CsA or FK506 on CSABP (CSABP-C) ATPase activity. The abscissa denotes CsA and FK506 concentrations (µM) and the ordinate denotes specific radioactivity of free ³²P.
[FIG. 8] A graph showing suppression of expression of CSABP by various siRNAs. The abscissa denotes relative expression percentage of CSABP (percentage relative to CSABP expression level in si-control-introduced cells).
[FIG. 9] A graph showing the effect of siRNA introducction on CSABP expression level. The ordinate denotes number of copies of CSABP gene in 12.5 ng of total RNA. Intact on the abscissa denotes cells that were treated only with a introduction reagent, si-control denotes cells that were treated with β-actin specific siRNA, and si-CSABP denotes cells that were treated with CSABP specific siRNA.
[FIG. 10] A graph showing the effect of siRNA introduction on HCV replication level. The ordinate denotes number of copies of HCV genome in 12.5 ng of total RNA. Intact on the abscissa denotes cells that were treated only with a introduction reagent, si-control denotes cells that were treated with β-actin specific siRNA, and si-CSABP denotes cells that were treated with CSABP specific siRNA.
[FIG. 11] A graph showing the effect of overexpression of CSABP in enhancing HCV replication. The ordinate denotes relative luciferase activity as an HCV genome replication index. With regard to the abscissa, Control denotes cells introduced only with pc-DNA3, CSABPfull 50 denotes cells introduced with 50 ng of CSABP-pcDNA3, and CSABPfull 150 denotes cells introduced with 150 ng of CSABP-pcDNA3.

[FIG. 12] A diagram showing the results of analysis by a GST pull-down method of association between CSABP and protein involved in HCV replication. 1/5 INP denotes a 1/5 amount of in vitro synthesized protein solution, GST denotes GST protein alone, and GST-CSABP denotes coculturing of GST-CSABP and an in vitro synthesized protein solution followed by subjecting a sample where GST-CSABP was recovered by glutathione Sepharose to SDS-PAGE, each in vitro synthesized protein being detected by autoradiography.
[FIG. 13] A diagram showing the results of analysis by a GST pull-down method of the effect of CsA on association between CSABP and NS5B or CyPB. 1/5 INP denotes a 1/5 amount of in vitro synthesized protein solution, GST denotes GST protein alone, and GST-CSABP denotes coculturing of GST-CSABP and an in vitro synthesis protein solution followed by subjecting a sample where GST-CSABP was recovered by glutathione Sepharose to electrophoresis using SDS-PAGE, each in vitro synthesized protein being detected by autoradiography.
[FIG. 14] A diagram showing the results of analysis by a GST pull-down method of association between CSABP and various fragments of NS5B. 1/5 INP denotes a 1/5 amount of in vitro synthesized protein solution, GST denotes GST protein alone, and GST-CSABP denotes coculturing of GST-CSABP and an in vitro synthesis protein solution followed by subjecting a sample where GST-CSABP was recovered by glutathione Sepharose to electrophoresis using SDS-PAGE, each in vitro synthesized protein being detected by autoradiography. Numerals in the diagram denote amino acid numbers of NS5B protein.
[FIG. 15] A photographic diagram showing the results of double staining of Huh7 cells or MH14 cells with anti-CSABP antibody and anti-KDEL antibody.

[FIG. 16] A photographic diagram showing the results of double staining of Huh7 cells or MH14 cells with anti-CSABP antibody and antibodies to various HCV-derived proteins.
[FIG. 17] A diagram showing the results of RNA binding assay for substances contained in MH14 cells. In the diagram, Whole cells denotes MH14 cell lysate, G denotes Sepharose on which an antibody specifically adsorbs, pU denotes a sediment binding to RNA, and r-CSABP denotes a recombinant CSABP protein.
[FIG. 18] A diagram showing the results of examination by RNA binding assay of the presence of CSABP-NS5B-CyPB trimer in vivo. In the diagram, Whole cells denotes MH14 cell lysate, G denotes Sepharose sediment on which an antibody specifically adsorbs, and pU denotes a sediment binding to RNA, which were subjected to electrophoresis using SDS-PAGE and western blotting with anti-CSABP antibody, anti-NS5B antibody, and anti-CyPB antibody.
[FIG. 19] A diagram showing the results of examination by RNA binding assay of the effect of deletion of CSABP on binding of NS5B and CyPB to RNA. In the diagram, Whole cells denotes MH14 cell lysate, G denotes Sepharose sediment on which an antibody specifically adsorbs, and pU denotes a sediment binding to RNA, which were subjected to electrophoresis using SDS-PAGE and western blotting with anti-CSABP antibody, anti-NS5B antibody, and anti-CyPB antibody.

[FIG. 20] A diagram showing the results of examination by RNA binding assay of the effect of CsA on binding of CSABP, NS5B, and CyPB to RNA. In the diagram, Whole cells denotes MH14 cell lysate, G denotes Sepharose sediment on which an antibody specifically adsorbs, and pU denotes a sediment binding to RNA, which were subjected to electrophoresis using SDS-PAGE and western blotting by anti-CSABP antibody, anti-NS5B antibody, and anti-CyPB antibody.
[FIG. 21] A graph showing the results of examination by luciferase reporter assay of transcription activity in various cDNA fragments in CSABP upstream region.
[FIG. 22] A diagram showing the position of a transcription factor binding motif in CSABP promoter region.
[FIG. 23] A graph showing the results of examination by luciferase reporter assay of transcription activity in cDNA fragments in which various mutations have been introduced into a transcription factor binding motif.
[FIG. 24] A diagram showing the results of analysis by CpG Island Searcher of CSABP promoter region. In the diagram, the bar in the vertical direction denotes CpG sequence position, and the band in the horizontal direction denotes CpG island region.
[FIG. 25] A diagram showing the results of examination of change in the expression level of CSABP with addition of 5-aza-2'-deoxycytidine. Numerals below the diagram denote 5-aza-2'-deoxycytidine concentration.
[FIG. 26] A diagram showing change in CSABP gene expression when human IL-1β, TNFα, or IL-6 was administered to normal human liver cells. The ordinate denotes percentage CSABP gene expression level when the control (Control, only medium administered) is defined as 100%.

### Best Mode for Carrying Out the Invention

The present invention relates to a composition for treating a CSABP-related disease, the composition containing a component that controls the abundance and/or function of a CSABP, and use of a component that controls the abundance and/or function of a CSABP in the production of a composition for treating a CSABP-related disease.
In the present invention, a CSABP means a substance, other than cyclophilin (CyP), calcineurin, and P-glycoprotein, that is present in humans or animals and binds to CsA, and typically denotes a protein having an amino acid sequence represented by SEQ ID No: 1 or a protein that a nucleic acid having a base sequence represented by SEQ ID No: 2 codes for (human CSABP). Furthermore, the CSABP in the present invention includes non-human animal CSABP that is homologous to human CSABP, for example, chimpanzee (XP_517881), bovine (XP_614159), dog (XP_531871), rat (XP_225959), mouse (XP_915830), Monodelphis domestica (XP_001370066), Xenopus tropicalis (AAI21455), or chicken (XP_413970) CSABP (NCBI database accession numbers being shown within parentheses). CSABP of animals other than those mentioned above may also be appropriately identified by searching a protein or nucleic acid database of an animal of interest based on the amino acid sequence represented by SEQ ID No: 1 or the base sequence represented by SEQ ID No: 2, and CSABP of various types of animals thus identified may also be used in the present invention. In the present description, amino acid numbers and base numbers for a CSABP are those of the above human CSABP unless otherwise specified.

As used herein, a CSABP may mean, depending on the context, in addition to the above-mentioned protein, a gene coding therefor or a CSABP variant. Furthermore, the above protein or gene is also called more specifically, for example, a 'CSABP protein', 'CSABP polypeptide', 'CSABP gene', 'CSABP nucleic acid', 'CSABP DNA', etc.
Naturally, among living individuals, there is a possibility of mutations occurring in a gene sequence or amino acid sequence that do not impair the physiological function of a protein, and CSABP in the present invention includes, in addition to proteins and nucleic acids having the same sequence as the sequence represented by the SEQ ID Nos above, those having sequences having one or more amino acids or bases, typically one or a few, for example 1, 2, 3, 4, 5, 6, 7, 8, 9, or 10 that are different from said sequences.

In the present invention, the abundance of a CSABP means, in addition to the mass or the number of moles of CSABP in a living body, the mass or the number of moles in a specific organ (e.g. liver, uterus, kidney, esophagus, lung, colon, breast, etc.), tissue (e.g. RNA virus target tissue, tumor tissue, etc.), a cell (e.g. an RNA virus target cell, tumor cell, etc.) and, furthermore, an organelle (e.g. endoplasmic reticulum, etc.). Furthermore, the abundance of a CSABP includes not only the abundance of the above-mentioned CSABP but also that of a substance having the same functions as the CSABP, for example, a CSABP functional variant. Therefore, controlling the abundance of CSABP means to absolutely or relatively increase, maintain, or decrease such an abundance of CSABP.
In the present invention, the functions of a CSABP include, for example, a function of binding to CsA or a derivative thereof, CyP, in particular CyPB, NS5B, and/or RNA, an RNA-dependent ATPase function, and an HCV replication promotion function. Therefore, controlling the function of a CSABP means promoting, maintaining, or suppressing at least one of these functions, and preferably all of these functions.

In the present invention, the component for controlling the abundance and/or the function of a CSABP includes any substance having such a function. Examples of components that enhance the abundance and/or the function of a CSABP include, but are not limited to, a CSABP, a CSABP functional variant, nucleic acids coding therefor and vectors containing such nucleic acids, and a substance that enhances CSABP gene expression; examples of components that reduce them include, but are not limited to, a CSABP dominant negative variant, an anti-CSABP antibody, NS5B, CyPB, and CSABP-binding variants thereof, nucleic acids coding therefor, vectors expressing these, and a substance that inhibits CSABP gene expression (e.g. a CSABP-targeting siRNA, ribozyme, antisense nucleic acid, or DNA/RNA chimera polynucleotide, vectors expressing these, etc.).

Examples of the CSABP functional variant include, but are not limited to, (1) a variant that has one or more mutations, typically one or a few, in the amino acid sequence represented by SEQ ID No: 1, but that still has equivalent functions to CSABP, (2) a variant that is coded by a nucleic acid having one or more mutations, typically one or a few, in a base sequence of a nucleic acid having the base sequence represented by SEQ ID No: 2 or a nucleic acid coding for the same polypeptide as said nucleic acid, and that has equivalent functions to CSABP, (3) a variant that is coded by a nucleic acid that hybridizes under stringent conditions with a complementary strand or a fragment thereof of a nucleic acid having the base sequence represented by SEQ ID No: 2, a nucleic acid coding for the same polypeptide as that of said nucleic acid, or a nucleic acid coding for the polypeptide of (2), and that has equivalent functions to CSABP, and (4) a variant that is coded by a nucleic acid having at least 60% homology to the base sequence represented by SEQ ID No: 2, preferably at least 70%, more preferably at least 80%, yet more preferably at least 90%, and particularly at least 95%, and that has equivalent functions to CSABP.

The variant may be appropriately prepared by any known method such as, for example, cleavage or insertion of a nucleic acid by a restriction enzyme, site-specific mutagenesis, or irradiation with radiation or UV rays.
Whether or not a given variant has equivalent functions to CSABP may be evaluated by, for example, analyzing binding to CsA or a derivative thereof, CyP, in particular CyPB, NS5B, and/or RNA, an RNA-dependent ATPase function, and/or an HCV replication promotion function, etc. by any known method, for example, in the case of binding a western blotting method, mass spectrometry, a pull-down method, a surface plasmon resonance (SPR) method, etc., in the case of RNA-dependent ATPase function a method by Okanami et. al., (Okanami et al., Nucleic Acids Res, 1998, 26: 2638-2643), and in the case of HCV replication various types of quantitative nucleic acid determination methods such as a PCR method, various types of quantitative protein determination methods such as EIA, ELISA, IRA, IRMA, and a western blotting method, etc. for HCV RNA or protein, and by comparing with an appropriate negative control or CSABP as a positive control. For example, with regard to a given variant, when at least one of the above-mentioned functions is superior to that of a negative control, for example, it is superior by 10% or greater, 25% or greater, 50% or greater, 75% or greater, or 100% or greater and/or when the function is 1/100 of that of CSABP or greater, 1/50 or greater, 1/25 or greater, 1/10 or greater, 1/5 or greater, or 1/2 or greater, this variant is included in the CSABP functional variants.

In the present invention, the CSABP dominant negative variant means a CSABP variant in which at least one CSABP function is lowered or deleted, and which can inhibit a function of wild type CSABP. Such a dominant negative variant may be selected from those into which a mutation to the wild type CSABP is introduced and for which any or all of the values measured for the CSABP functions in the same manner as for the above-mentioned functional variants are smaller than those of the wild type CSABP, and is more preferably selected from those that lower the function of the wild type CSABP when they are made to coexist with the wild type CSABP. With regard to sites into which a mutation is introduced, for example, the 191st to 378th amino acids, in which the RNA helicase motif is present, and its vicinity, the 940th to 945th amino acids, which is a CsA-binding region, and its vicinity, etc. can be cited.

In the present invention, the anti-CSABP antibody includes any antibody targeting a CSABP, and various functional fragments such as Fab, Fab', F(ab')₂, scFv, dsFv (disulfide stabilized V region fragment), and CDR-containing fragments of such an antibody. The anti-CSABP antibody may appropriately be prepared by any known method such as, for example, a method in which an animal (rabbit, goat, rat, mouse, etc.) is immunized with CSABP protein or a fragment thereof, serum is collected after a predetermined period of time, and a polyclonal antibody is obtained, or a method in which lymphocytes, in particular B cells, are collected from an immunized animal after a predetermined period of time, these are fused with immortal cells such as a tumor cell line to give a hybridoma, and this is further cultured to give a monoclonal antibody. The antibody may be subjected to various modifications according to the biological species of the subject so that an unnecessary immunoreaction in the subject to which it is administered is not caused. For example, by fusing a variable region of a non-human biological species with a human constant region a human type chimera antibody may be obtained, and by grafting CDR of a non-human biological species into an appropriate position of a human antibody a human type CDR-grafted antibody may be obtained. A humanized antibody that has been thus modified for the purpose of application to humans and, moreover, a modified antibody for application to various non-human animals by the same method are also included in the anti-CSABP antibody used in the present invention.

In the present invention, NS5B includes, in addition to one derived from HCV (NP_751928), flaviviridae viruses such as hog cholera virus (NP_777506), GB virus A (NP_803217), GB virus B (NP_757361), GB virus C (NP_803209), bovine viral diarrhea virus (NP_777493, NP_776271), and border disease virus (NP_777545), and in particular those derived from viruses of the pestivirus genus and the hepacivirus genus (NCBI database accession numbers are given in parentheses). Among them, NS5B that is derived from a virus as a target for treatment or detection is preferable.
In the present invention, CyPB includes, in addition to human-derived ones (AAH32138, AAH20800, AAH08848, AAH08848), those derived from rat (NP_071981), mouse (NP_035279), cattle (NP_776577), chicken (NP_990792), etc. (NCBI database accession numbers are given in parentheses). Among them, CyPB that is derived from a subject for treatment or diagnosis is preferable.

In the present invention, the CSABP-binding variant of NS5B or CyPB means a variant that has one or more amino acid mutations from the above NS5B or CyPB but maintains binding properties to CSABP. For example, since CSABP binds to a portion comprising the 1st to 200th amino acids and a portion comprising the 401st to 520th amino acids of HCV NS5B (see example 7 (1)), an NS5B fragment containing at least one of these portions is included in the CSABP-binding variants. Among the above-mentioned variants, one that can inhibit the function of CSABP is preferable, and examples of such a variant include an NS5B variant that contains a CSABP-binding site but does not contain a CyPB binding site (in the case of HCV, the 521st to 591st amino acids) and an NS5B variant that contains a CSABP-binding site but does not contain a RNA polymerase region (in the case of HCV, the 116th to 398th amino acids).

The nucleic acids in the present invention include various polymers such as DNA, RNA, a DNA/RNA hybrid, and PNA.
The term 'stringent conditions' used herein is a well-known parameter in the present technical field and is described in standard protocol handbooks such as, for example, Sambrook et al., Molecular Cloning: A Laboratory Manual, 3rd ed., Cold Spring Harbor Press (2001) and Ausubel et al., Current Protocols in Molecular Biology, Greene Publishing Associates (1992).
The stringent conditions in the present invention denote, for example, hybridization at 65°C by a hybridization buffer containing 3.5×SSC (0.15 M sodium chloride/0.15 M sodium citrate, pH 7), Ficoll 0.02%, polyvinylpyrrolidone 0.02%, bovine serum albumin 0.02%, NaH₂PO₄ 25 mM (pH 7), SDS 0.05%, and EDTA 2 mM. After hybridization, a membrane to which DNA is transferred is washed with 2xSSC at room temperature and subsequently with 0.1 to 0.5×SSC/0.1×SDS at a temperature up to 68°C. Alternatively, stringent hybridization may be carried out using a commercial hybridization buffer such as ExpressHyb™ Hybridization Solution (Clontech) under hybridization and washing conditions described by the manufacturer.
There are other conditions, reagents, etc. that can be employed and result in the same level of stringency, and since it is expected that a person skilled in the art will be conversant with such conditions, they are not specifically described herein. However, it is possible to modify the conditions so as to clearly identify a nucleic acid coding for a CSABP variant or a CSABP promoter homologue or allele.

With regard to an siRNA, ribozyme, antisense nucleic acid, and DNA/RNA chimera polynucleotide that target CSABP, those prepared by any known method may be used.
Examples of the siRNA include those described in SEQ ID Nos: 9 to 14, but the siRNA that can be used in the present invention is not limited thereto, and includes one corresponding to any portion of SEQ ID No: 2.
Furthermore, JP-A-2003-219893 describes a double-stranded polynucleotide, consisting of DNA and RNA, that inhibits expression of a target gene. This polynucleotide may be a DNA/RNA hybrid in which one of the two strands is DNA and the other is RNA or a DNA/RNA chimera in which one part of a strand is DNA and the other part of the same strand is RNA. Such a polynucleotide preferably comprises 19 to 25 nucleotides, more preferably 19 to 23 nucleotides, and yet more preferably 19 to 21 nucleotides; in the case of a DNA/RNA hybrid it is preferable for the sense strand to be DNA and for the antisense strand to be RNA, and in the case of a DNA/RNA chimera it is preferable for one part on the upstream side of a double-stranded polynucleotide to be RNA. With regard to such polynucleotides, it is possible to prepare one having any sequence in accordance with a standard procedure by a chemical synthesis method known per se.

The above-mentioned nucleic acid may be used as a naked nucleic acid as it is or may be carried by various vectors. As a vector, any known vector such as a plasmid vector, a phage vector, a phagemid vector, a cosmid vector, or a virus vector may be used. The vector preferably contains at least a promoter for enhancing expression of the nucleic acid carried thereon, and in this case said nucleic acid is preferably operably linked to such a promoter. A nucleic acid being operably linked to a promoter means that said nucleic acid and said promoter are positioned so that a protein coded by said nucleic acid is appropriately produced by the operation of the promoter. The vector may or may not be able to replicate within the host cell, and gene transcription may be carried out outside or inside the nucleus of the host cell. In the case of the latter, the nucleic acid may be introduced into the genome of the host cell.

In the present invention, a substance that controls CSABP gene expression includes any substance having such a function.
As used herein, 'expression' of a gene or protein includes various stages via which a polypeptide is synthesized from a gene, such as gene transcription, splicing, translation, and post-translational modification, and 'controlling expression' therefore means controlling, that is, enhancing or suppressing, at least one of the steps such as gene transcription, splicing, translation, and post-translational modification.
Examples of substances that enhance CSABP gene expression include, but are not limited to, inflammatory cytokines (IL-1 such as IL-1α or IL-1β, IL-5, IL-6, IL-12, IL-15, IL-17, IL-18, TNF such as TNFα, etc.), transcription factors belonging to the CREB/ATF family or the GATA family (CREB, ATF1 to ATF6, GATA1 to GATA6, etc.), transcription factor AP-1, functional equivalents thereof, and substances that enhance the abundance and/or the function of said transcription factors (PKA, cAMP, adenylate cyclase, p90^{RSK}, ERK, p38, MAPKAPK-2, MSK-1, CaMK, calcium ion, Akt/PKB, JNK, FRK (Fos-related kinase), RSK (ribosomal S6 kinase), casein kinase II, GSK-3β, and functional equivalents thereof, etc.).

On the other hand, examples of components that suppress CSABP gene expression include, but are not limited to, CSABP-targeting siRNA, ribozyme, antisense nucleic acid, and DNA/RNA chimera polynucleotide, vectors expressing same, and inhibitors for transcription factors belonging to the CREB/ATF family or the GATA family or transcription factor AP-1 such as, for example, a substance, preferably specifically, binding to a CSABP gene promoter region (bases -463 to -1 (SEQ ID No: 37) when A of initiation codon ATG of said gene is defined as base +1 and the base one base upstream of ATG (5' side) is defined as base -1, preferably bases -463 to -44 (SEQ ID No: 30)), particularly a transcription factor binding site thereof (DNA portion containing at least one of bases -220 to -213: (CREB binding site: TGACGTCA), bases -188 to -179 (GATA binding site: CGTGATATCA), and bases -150 to -140 (AP-1-binding motif: GGTGACTGAGG)) (e.g. a nucleic acid hybridizing with the above portion, etc.), a dominant negative variant of the above transcription factor (e.g. CREBM1 (Gonzalez, G. A., and Montminy, M. R. (1989) Cell 59, 675-680), KCREB (Reusch, J. E. et al. (2000). Mol. Cell. Biol. 20, 1008-1020.), DN CREB (Nguyen, L. Q. et al. (2000) Mol. Endocrinol. 14, 1448-1461), A-CREB (Ahn S. et al. (1998) Mol Cell Biol 18: 967-977)), antibodies to said transcription factors, nucleic acids coding therefor, vectors containing said nucleic acids, and substances that inhibit activation of said transcription factors (e.g. PKA, cAMP, adenylate cyclase, p90^{RSK}, ERK, p38, MAPKAPK-2, MSK-1, CaMK, calcium ion, Akt/PKB, JNK, FRK, RSK, casein kinase II, and GSK-3β inhibitors, for example, antibodies to these substances, PKA inhibitors such as H89 and Rp-8-Br-cAMP, ERK pathway inhibitors such as PD98059, U0126, and FR180204, p38 inhibitors such as SB203580, SB202190, and SB239063, CaMK inhibitors such as KN-93, KN-62, AIP, CaM Kinase II Inhibitor 281-301, lavendustin C, K252a, rottlerin, ML-7, ML-9, STO-609, W-7, and W-5, intracellular calcium movement inhibitors such as BAPTA-AM, A23187, and ionomycin, etc.).
Furthermore, as a substance that controls CSABP gene expression, a substance that has been identified by a method described later for screening a substance that controls CSABP gene expression may be used.

Preferred substances that control CSABP gene expression in the present invention are CSABP-targeting siRNA, ribozyme, antisense nucleic acid, and DNA/RNA chimera polynucleotide, vectors expressing these, substances that enhance CSABP gene expression such as transcription factors belonging to the CREB/ATF family (CREB, ATF1 to ATF6, and functional equivalents thereof, in particular CREB), and substances that enhance the abundance and/or the function of said transcription factors (e.g. PKA, cAMP, adenylate cyclase, p90^{RSK}, ERK, p38, MAPKAPK-2, MSK-1, CaMK, calcium ion, Akt/PKB, and functional equivalents thereof, etc.), and substances that suppress CSABP gene expression such as inhibitors for transcription factors belonging to the CREB/ATF family, including substances that, preferably specifically, bind to a CSABP gene promoter region (bases -463 to -1 (SEQ ID No: 37), preferably bases -463 to -44 (SEQ ID No: 30), in particular a portion of DNA containing a CREB binding site (bases -220 to -213: TGACGTCA)) (e.g. a nucleic acid hybridizing with said portion, etc.), dominant negative variants of the above-mentioned transcription factors (e.g. CREBM1, KCREB, DN CREB, A-CREB), antibodies to said transcription factors, nucleic acids coding therefor, vectors containing said nucleic acids, and substances that inhibit activation of said transcription factors (e.g. PKA, cAMP, adenylate cyclase, p90^{RSK}, ERK, p38, MAPKAPK-2, MSK-1, CaMK, calcium ion, Akt/PKB, etc. inhibitors, for example, antibodies to these substances, PKA inhibitors such as H89 and Rp-8-Br-cAMP, ERK pathway inhibitors such as PD98059, U0126, and FR180204, p38 inhibitors such as SB203580, SB202190, and SB239063, CaMK inhibitors such as KN-93, KN-62, AIP, CaM Kinase II Inhibitor 281-301, lavendustin C, K252a, rottlerin, ML-7, ML-9, STO-609, W-7, and W-5, intracellular calcium movement inhibitors such as BAPTA-AM, A23187, and ionomycin, etc.).
Among the above-mentioned substances that control CSABP gene expression, those that are polypeptides include nucleic acid molecules coding therefor and vectors containing same, and those that are polynucleotides include vectors containing same.

In the present invention, the CSABP-related disease includes a disease for which CSABP is involved in onset, progression, remission, cure, etc., a disease characterized by expression of CSABP, etc. Examples of diseases for which CSABP is involved in onset, progression, remission, cure, etc. include RNA virus infection, typically positive strand RNA virus infection, and more typically flaviviridae virus infection. Therefore, such diseases include reovirus infection, SARS, torovirus infection, yellow fever, Japanese encephalitis, dengue fever, west Nile fever, bovine viral diarrhea-mucosal disease, hog cholera, border disease, hepatitis C, foot and mouth disease, polio (poliomyelitis), hepatitis A, common cold, rubella, sindbisvirus infection, equine viral arteritis, simian hemorrhagic fever, norovirus infection, sapovirus infection, rabbit hemorrhagic fever, feline calicivirus disease, vesicular exanthema of swine, hepatitis E, Marburg disease, Ebola hemorrhagic fever, rabies, vesicular stomatitis, measles, pseudorinderpest of small ruminants, canine distemper, rinderpest, mumps, menangle virus infection, Newcastle disease, RS virus infection, human/metapneumovirus infection, Nipah virus infection, influenza, Akabane disease, hantavirus pulmonary syndrome, hemorrhagic fever with renal syndrome, lymphocytic choriomeningitis, lassa fever, Argentine hemorrhagic fever, Bolivian hemorrhagic fever, AIDS, adult T cell leukemia, etc., more typically yellow fever, Japanese encephalitis, dengue fever, west Nile fever, bovine viral diarrhea-mucosal disease, hog cholera, border disease, hepatitis C, etc., and particularly bovine viral diarrhea-mucosal disease, hog cholera, border disease, hepatitis C, etc.
Furthermore, among RNA virus infections, those accompanied by inflammation and/or those following inflammation, and those accompanied by release of inflammatory cytokine and/or those following release of inflammatory cytokine are also suitable.

Examples of diseases characterized by expression of CSABP include, in addition to the above-mentioned RNA virus infection, neoplastic diseases such as, for example, tumor of the liver, uterus, kidney, esophagus, lung, colon, breast, etc., typically liver cancer, uterine cancer, kidney cancer, esophageal cancer, lung cancer, colon cancer, breast cancer, etc. and, furthermore, inflammatory diseases, particularly diseases accompanied by the release of inflammatory cytokines such as, for example, encephalitis, conjunctivitis, keratitis, uveitis, retinitis, rhinitis, sinusitis, otitis, stomatitis, gingivitis, esophagitis, gastritis, enteritis, hepatitis, pharyngitis, laryngitis, tracheitis, bronchitis, pneumonia, cystitis, ureteritis, urethritis, nephritis, vaginitis, uteritis, ovaritis, orchitis, vasculitis, pericarditis, neuritis, myositis, osteitis, periostitis, osteomyelitis, chondritis, meningitis, arthritis, or dermatitis.
A disease characterized by expression of CSABP also includes a state in which replication of an RNA virus is promoted. Such a state is caused by, for example, inflammatory cytokines such as TNFα acting on normal cells to thus induce expression of CSABP in the cells. When this state is left as it is, among such cells in which expression of CSABP has been induced, cells that have not been infected with RNA virus and are then infected with RNA virus or cells that have already been infected with RNA virus are provided with an environment in which said virus easily replicates, and RNA virus infection develops or becomes severe. Therefore, by administering the composition of the present invention to thus inhibit expression of CSABP in cells in such a state, it becomes possible to prevent the onset of RNA virus infection or suppress its aggravation.
The presence or absence of CSABP expression may be confirmed appropriately by an appropriate detection method known to a person skilled in the art by the use of a component, described later, that detects a CSABP-coding nucleic acid or an expression product thereof.
Furthermore, the CSABP-related disease includes various diseases that follow the above-mentioned diseases such as, for example, hepatic fibrosis, hepatic cirrhosis, and liver cancer, which follow hepatitis C, in particular chronic hepatitis C.
In order to make the composition of the present invention reach a desired organ or cell efficiently, the composition may be targeted thereat. Targeting may employ any known method, and examples thereof include modification by a ligand or an antibody to a molecule that is specifically expressed in a desired organ or cell. In particular, by making the active component of the present composition be carried on a carrier described in WO2006/068232, it may be delivered specifically to the liver.
Furthermore, with regard to the composition of the present invention, in order to promote delivery of the active component to the interior of a cell, the component may be contained in an intracellular delivery carrier such as a liposome, in particular a cationic liposome, may be bound to the carrier, or may be bound to a substance that promotes intracellular delivery such as a TAT peptide.

The composition for treatment of a CSABP-related disease of the present invention may contain, in addition to a component that controls the abundance and/or the function of CSABP, another component that is effective for the treatment of a CSABP-related disease, or may be used in combination with another drug that is effective for the treatment of a CSABP-related disease.
Specifically, when it is intended to treat an RNA virus infection, for example, an antiviral drug such as oseltamivir, zanamivir, amantadine, rimantadine, azidothymidine, didanosine, zalcitabine, sanilvudine, lamivudine, abacavir, tenofovir, emtricitabine, lamivudine, tenofovir, emtricitabine, nevirapine, efavirenz, delavirdine, indinavir, saquinavir, ritonavir, nelfinavir, amprenavir, lopinavir, atazanavir, fosamprenavir, enfuvirtide, adefovir, or entecavir may be used.
Furthermore, when the RNA virus infection is one accompanied by inflammation and/or one following inflammation or is one accompanied by release of inflammatory cytokine and/or one following release of inflammatory cytokine, a drug that suppresses inflammation and/or release of inflammatory cytokine such as, for example, various steroid type antiinflammatory drugs, nonsteroidal antiinflammatory drugs, a substance such as an siRNA or antisense nucleic acid that suppresses expression of inflammatory cytokine, and/or a drug that suppresses the action of inflammatory cytokine such as, for example, an antibody to inflammatory cytokine or an inflammatory cytokine receptor antagonist may be contained in the composition of the present invention or may be used in combination therewith.

In particular, when it is intended to treat hepatitis C, for example, interferons (IFNα, IFNβ, IFNβ-1a (Rebif), IFNγ, IFNω, Infergen, Albuferon, Multiferon, Locteron, Belerofon, etc.), pegylated interferons (PegIFNα-2a (Pegasys), PegIFNα-2b (PegIntron)), ribavirin, CsA and derivatives thereof (e.g. NIM811, modified cyclosporin described in Patent Document 2, etc.), amantadine, Medivir HCV Protease, AB68, PYN17, ITMN-191, A-831, Oglufanide, R7128, nitrazoxanide, Bavituximab, NOV-205, XTL-2125, AVI-4065, ANA975, HCV-796, mitoquinone (MitoQ), ACH-0137171, XTL-6865, DEBIO-025, UT-231B, R1626, G126270, SCH503034 (Boceprevir), VX950 (Telaprevir), PF-03491390, Civacir, methylene blue (Suvus), celgosivir, VGX-410C, valopicitabine, JBK-122, taribavirin (Viramidine), EMZ702, ChronVac-C, TG4040, PeviPROTM, GGI-5005, HCV/MF59, IC41, INNO0101 (E1), NA255, lovastatin, a cycloalkyl heterocyclic compound described in Patent Document 1, etc. may be used.

When it is intended to treat hepatic fibrosis or hepatic cirrhosis, which accompany hepatitis C, for example, a TGFβ activity inhibitor such as a truncated TGFβII receptor or a soluble TGFβII receptor, a growth factor preparation such as HGF, an MMP production promoter such as an MMP gene-containing adenovirus vector, a hepatic stellate cell activation/growth inhibitor including PPARγ ligand, an angiotensin II type I receptor antagonist, a PDGF tyrosine kinase inhibitor, a sodium channel inhibitor such as amiloride, an apoptosis inducer such as compound 861 or gliotoxin, a substance having an effect in suppressing expression of a target molecule, such as siRNA, ribozyme, or antisense nucleic acid (including RNA, DNA, PNA, or a complex thereof) targeting an extracellular matrix component such as collagen, proteoglycan, tenascin, fibronectin, thrombospondin, osteopontin, osteonectin, or elastin for suppressing production of these components, a substance having a dominant negative effect such as a dominant negative variant of these components, a vector expressing same, a drug for suppressing formation of collagen, such as corticosteroid, penicillamine, colchicine, interferon γ, or a prostaglandin derivative, etc. may be used.

Moreover, when it is intended to treat a tumor, for example, an alkylating agent such as ifosfamide, nimustine hydrochloride, cyclophosphamide, dacarbazine, melphalan, or ranimustine, an antimetabolite such as gemcitabine hydrochloride, enocitabine, cytarabine ocfosfate, a cytarabine preparation, tegafur/uracil, a tegafur/gimeracil/oteracil potassium combination drug, doxifluridine, hydroxycarbamide, fluorouracil, methotrexate, or mercaptopurine, an antitumor antibiotic such as idarubicin hydrochloride, epirubicin hydrochloride, daunorubicin hydrochloride, doxorubicin hydrochloride, pirarubicin hydrochloride, bleomycin hydrochloride, peplomycin sulfate, mitoxantrone hydrochloride, or mitomycin C, an alkaloid such as etoposide, irinotecan hydrochloride, vinorelbine tartarate, docetaxel hydrate, paclitaxel, vincristine sulfate, vindesine sulfate, or vinblastine sulfate, a hormone therapy agent such as anastrozole, tamoxifen citrate, toremifene citrate, bicalutamide, flutamide, or estramustine phosphate, a platinum complex such as carboplatin, cisplatin, or nedaplatin, an angiogeneis inhibitor such as thalidomide, neovastat, or bevacizumab, L-asparaginase, etc. may be used.

In the present invention, the term 'treatment' includes all types of medically allowed preventive and/or therapeutic interventions for the purpose of cure, temporary remission, prevention, etc. of a disease. For example, when the disease is hepatitis C, the term 'treatment' includes medically allowed interventions for various purposes such as delaying or halting progression of hepatitis C, regression or elimination of lesions, and preventing onset or recurrence of hepatitis C.
Furthermore, the term 'subject' in the present invention means any living individual, preferably an animal, more preferably a mammal, and yet more preferably a human individual. In the present invention, the subject may be healthy or may be affected with any disease, but when it is intended to treat a CSABP-related disease, typically the subject means a subject that is affected with said disease or has a high risk of being affected such as, for example, chicken, mouse, rat, cat, sheep, goat, cattle, horse, pig, monkey, or human.

The above-mentioned composition for treatment of a CSABP-related disease may be administered via various routes including both oral and parenteral routes; examples thereof include, but are not limited to, oral, intravenous, intramuscular, subcutaneous, local, rectal, intraarterial, intraportal, intraventricular, transmucosal, percutaneous, nasal, intraperitoneal, intrapulmonary, intrauterine, etc. routes, and a preparation may be formed into a dosage form that is suitable for each administration route. Such a dosage form and a formulation method may employ any known form and method as appropriate (see e.g. 'Hyoujun Yakuzaigaku' (Standard Pharmaceutics), Ed Y. Watanabe et al., Nankodo, 2003, etc.).
For example, examples of a dosage form suitable for oral administration include, but are not limited to, powder, granule, tablet, capsule, liquid, suspension, emulsion, gel, and syrup, and examples of a dosage form suitable for parenteral administration include injections such as injectable solution, injectable suspension, injectable emulsion, and a ready-to-use injection. A preparation for parenteral administration may be in the form of an aqueous or nonaqueous isotonic sterile solution or suspension. Specifically, for example, it may be formulated into an appropriate unit administration form by appropriate combination with a pharmacologically acceptable carrier or medium, specifically, sterile water, physiological saline, vegetable oil, an emulsifier, a suspending agent, a surfactant, a stabilizer, an excipient, a vehicle, an antiseptic agent, a binder, etc. The amount of active component in these preparations may be appropriately set so that a dose that is effective for treatment can be supplied to a subject by an assumed number of administrations.

Examples of an aqueous solution for injection include physiological saline, an isotonic solution containing glucose or another adjuvant, such as for example D-sorbitol, D-mannose, D-mannitol, or sodium chloride, and it may be used in combination with an appropriate dissolution adjuvant such as, for example, an alcohol, specifically ethanol or a polyalcohol such as, for example, propylene glycol or polyethylene glycol, a nonionic surfactant such as, for example, polysorbate 80 or HCO-50, etc.
Examples of an oily liquid include sesame oil and soybean oil, and it may be used in combination with a dissolution adjuvant such as benzyl benzoate or benzyl alcohol. Furthermore, it may be mixed with a buffer agent such as for example a phosphoric acid buffer or a sodium acetate buffer, a soothing agent such as procaine hydrochloride, a stabilizer such as, for example, benzyl alcohol or phenol, an antioxidant, etc. A prepared injection is usually charged into an appropriate container such as an ampoule, a vial, a tube, a bottle, or a pack.

Administration of the present composition into a subject body may be via any of the above-mentioned routes, but parenteral administration is preferable, local administration or intravenous administration is more preferable, and intraportal or intratumoral administration is particularly preferable. The number of administrations is preferably 1, but administration may be carried out a plurality of times depending on conditions. Furthermore, the duration of administration may be a short time or it may be a long-term continuous administration. More specifically, the composition of the present invention may be administered via injection or percutaneously. Examples of administration via injection include, but are not limited to, local injection, intravenous injection, intraarterial injection, selective intraarterial infusion, intraportal infusion, intramuscular injection, intraperitoneal injection, subcutaneous injection, intratumoral injection, intrathecal injection, intraarticular injection, and intracerebroventricular injection. In the case of intravenous injection, administration is possible by a usual blood transfusion procedure, and since it is unnecessary to carry out surgery on a subject or to subject it to local anesthetic, the burden on both the subject and the practitioner can be alleviated. It is also advantageous since administration is possible outside an operating theater.

Furthermore, the present invention relates to a method for treating a CSABP-related disease, the method including administering to a subject an effective dose of the composition for treatment of a CSABP-related disease of the present invention. In the treatment method of the present invention, administration of the composition for treatment of the present invention to a subject may be carried out suitably in accordance with, for example, the above-mentioned administration method. Moreover, a physician or a veterinarian can administer to a subject the agent of the present invention by appropriately modifying the above-mentioned administration method. The effective dose referred to here is a dose that suppresses onset of a CSABP-related disease, alleviates symptoms, or prevents progression, and is preferably a dose that prevents onset of a CSABP-related disease or cures a CSABP-related disease. Furthermore, it is preferably a dose that does not cause an adverse effect that exceeds the benefits of administration. Such a dose may be appropriately determined by an in vitro test using cultured cells, etc. or a test using a model animal such as a mouse, a rat, a dog, or a pig.

In the treatment method of the present invention, since a specific dose of the composition for treatment of a CSABP-related disease to be administered is determined while taking into consideration various conditions relating to the subject to be treated such as, for example, severity of symptoms, general health state of the subject, age, weight, gender of the subject, diet, the timing and frequency of administration, a pharmaceutical used in combination, response to the treatment, and compliance with the treatment, it might be different from a general effective dose, but in such a case these methods are still included in the scope of the present invention.
Examples of the administration route include various routes covering both oral and parenteral such as, for example, oral, intravenous, intramuscular, subcutaneous, local, intratumoral, rectal, intraarterial, intraportal, intraventricular, transmucosal, percutaneous, nasal, intraperitoneal, intrathecal, intraarticular, intracerebroventricular, intrapulmonary, and intrauterine routes.
The frequency of administration varies depending on the properties of the composition used and the condition of the subject as described above but it may be, for example, a plurality of times per day (that is, 2, 3, 4, 5, or more times per day), once a day, every few days (that is, every 2, 3, 4, 5, 6, or 7 days, etc.), once a week, once every few weeks (that is, once every 2, 3, or 4 weeks, etc.).
Furthermore, in the treatment method of the present invention, in addition to the composition for treatment of a CSABP-related disease of the present invention, another drug that is effective for the treatment of a CSABP-related disease described above may also be used in combination.

The present invention also relates to a composition for diagnosing a CSABP-related disease, the composition containing a component that detects a CSABP-coding nucleic acid or an expression product thereof.
In the present invention, as a component that detects a CSABP-coding nucleic acid or an expression product thereof, any substances that bind to a CSABP-coding nucleic acid or an expression product thereof may be used. Examples of such a substance include CSABP-binding substances such as an anti-CSABP antibody, CsA and a derivative thereof, NS5B, CyPB, and a CSABP-binding variant thereof, and nucleic acids specifically hybridizing with a CSABP-coding nucleic acid, a unique fragment thereof, a transcription product (e.g. mRNA) or a splice product of said nucleic acid. The unique fragment of the CSABP-coding nucleic acid referred to means, of the genome, a fragment that is present only in the CSABP-coding nucleic acid, and is typically a fragment having a 12 to 32 base length (e.g. 12, 13, 14, 15, 16, 17, 18, 19, 20, 21, 22, 23, 24, 25, 26, 27, 28, 29, 30, 31, and 32 base length) or longer. The CSABP-binding variant of NS5B or CyPB is preferably a variant that binds to CSABP but lacks other physiological activities. Examples of such a variant include an NS5B variant that contains a CSABP-binding site but does not contain a CyPB binding site (in the case of HCV, the 521st to 591st amino acids) and an NS5B variant that contains a CSABP-binding site but does not contain an RNA polymerase region (in the case of HCV, the 116th to 398th amino acids).

The detecting component may have attached thereto various labels for convenience of detection. The label may be any that is known and may be selected from, for example, any radioisotopes, magnetic substances, substances binding to the above-mentioned component (e.g. an antibody), biotin, fluorescent substances, fluorophores, chemiluminescent substances, and enzymes.
For detection, any known method may be used. Examples of such a method include, but are not limited to, an immunoprecipitation method utilizing an anti-CSABP antibody, EIA, ELISA, IRA, IRMA, a western blotting method, an immunohistochemical method, an immunocytochemical method, an enzyme chromogenic method, fluorescence detection method, radioactivity measurement method, autoradiographic method, flow cytometric method, tissue staining method, and cell staining method, which utilize a labeled CSABP-binding substance (CsA, a derivative thereof, NS5B, CyPB, and a CSABP-binding variant thereof, etc.), various hybridization methods, northern blot method, Southern blot method, which utilize a nucleic acid specifically hybridizing with a CSABP-coding nucleic acid, a unique fragment thereof, a transcription product of said nucleic acid (e.g. mRNA), or a splice product, and various PCR methods.

A sample used for the above-mentioned detection includes, for detection of a CSABP protein, for example, blood, a mucosal scraping, and various cells or organs collected by surgery, a biopsy, etc. The sample may be subjected to a detection operation as it is, but if, for example, cells are disrupted by sonication, etc. and a cell extract is prepared from the centrifugal supernatant, the detection operation becomes easy. Furthermore, when a CSABP-coding nucleic acid is to be detected, for example, blood, saliva, oral mucosa, blister contents, a skin fragment containing a blister, feces, urine, various cells, body fluids, organs, etc. collected by surgery, a biopsy, etc. may be used as a sample. These samples may be subjected to detection as they are, but it is preferable to subject them to extraction/purification of DNA and/or RNA in advance. An extraction method for DNA or RNA may be selected from any method known in the art; for extraction of DNA, for example, a method such as a proteinase K/phenol extraction method, a proteinase K/phenol/chloroform extraction method, an alkaline lysis method, or a boiling method may be used, and for extraction of RNA, for example, a method such as a guanidine acid phenol (AGPC) method or an SDS-phenol method may be used. DNA and/or RNA may be amplified prior to detection. For amplification, any known method including a PCR method may be used. By amplifying DNA or RNA, the detection operation becomes easy.

In the present invention, a CSABP-related disease is diagnosed based on the presence/absence or abundance of a CSABP or a nucleic acid coding therefor. For example, contraction of a disease characterized by expression of a CSABP may be diagnosed by detecting the expression of a CSABP or a nucleic acid coding therefor. Furthermore, with regard to a disease for which the expression of a CSABP increases/decreases accompanying the progression/regression thereof, by monitoring changes in the amount of expression of CSABP or nucleic acid coding therefor the progression or regression thereof and, furthermore, remission or cure thereof may be diagnosed.
Therefore, the present invention also relates to a method for diagnosing a CSABP-related disease, the method including a step of detecting the presence of, or a step of measuring an abundance of a CSABP or a nucleic acid coding therefor in a sample collected from a subject. The subject, the sample, and the measurement method in this method are as described above.

The present invention also relates to a method for screening a component that detects and/or controls a CSABP, the method including a step of making a test substance coexist with a CSABP or a fragment thereof. The component that controls a CSABP referred to here typically means a component that controls the abundance and/or the function of a CSABP.
The test substance used in the method of the present invention is not particularly limited and includes a polymer such as a protein and various low molecular weight compounds. The test substance may be a known substance or may be a novel substance that is synthesized by any known method such as recombinatorial chemistry.
The CSABP fragment in the method of the present invention may be any portion of a CSABP, but is preferably a portion involved in the function of the CSABP, for example, a portion containing the entirety or part of the DExDc domain (191st to 378th amino acids), which is involved in RNA helicase activity. Such a fragment may be obtained by any known method such as digestion by a CSABP protein proteinase such as, for example, trypsin or papain, digestion of a CSABP gene by restriction enzyme, or amplification of a predetermined portion of a CSABP gene by a PCR method using a specific primer, etc.
The test substance or CSABP may be immobilized on any solid support. Examples of the support include, but are not limited to, plates, beads, and chips made of a material such as glass, nitrocellulose, nylon, or plastic. A plurality of types of test substances may be tested all at once by immobilizing a plurality of types of test substances on chips, etc. and making them contact labeled or unlabeled CSABP or by immobilizing a plurality of CSABPs and making them contact a plurality of types of test substances.

Contacting a test substance with a CSABP or a fragment thereof may be achieved by any method, but it is typically carried out in an inactive medium, and preferably in an inactive liquid medium. The inactive medium may employ any known medium that does not cause a reaction of the test substance or CSABP, such as for example distilled water, physiological saline, or PBS.
In one embodiment of the method of the present invention, the binding properties (affinity) between a test substance and a CSABP or a fragment thereof is used as an indicator for screening. Measurement of binding properties (dissociation constant, association constant, etc.) may be carried out by any known method, for example, a surface plasmon resonance (SPR) method, an equilibrium dialysis method, an atomic force microscopic method (Florin EL et al., Science. 1994; 264 (5157): 415-7), various competitive binding assays in which a test substance is made to compete with a known CSABP-binding substance (CsA, NS5B, CyPB, anti-CSABP antibody, etc.) labeled with a radioisotope, an enzyme, a fluorochrome, etc. With regard to a preferred dissociation constant (K_{d}) in the present invention, it is for example 10⁻⁶ M or less, preferably 10⁻⁷ M or less, more preferably 10⁻⁸ M or less, and yet more preferably 10⁻⁹ M or less, and with regard to a preferred association constant (Kₐ), it is 10⁶ M⁻¹ or greater, preferably 10⁷ M⁻¹ or greater, more preferably 10⁸ M⁻¹ or greater, and yet more preferably 10⁹ M⁻¹ or greater. A test substance having high binding properties may preferably be used as a component that detects and/or inhibits CSABP.

In another embodiment of the method of the present invention, promotion or suppression of the function of a CSABP by the co-existence of a test substance is used as an indicator for screening. Examples of the function of a CSABP include the above-mentioned binding properties to CsA or a derivative thereof, CyP, in particular CyPB, NS5B and/or RNA, and the RNA-dependent ATPase function and/or the HCV replication promotion function, and changes in these functions of a CSABP in the presence of a test substance are analyzed by any known method, for example, in the case of binding properties, by a western blotting method, mass spectrometry, a pull-down method, a surface plasmon resonance (SPR) method, etc., in the case of the RNA-dependent ATPase function, by the above-mentioned method by Okanami et al., etc., in the case of the HCV replication promotion function, by various quantitative nucleic acid determination methods such as a PCR method for HCV RNA or various quantitative protein determination methods such as EIA, ELISA, IRA, IRMA, or a western blotting method for HCV protein. A substance that promotes the function of CSABP thus identified may be used preferably as a component that promotes the function of a CSABP, and a substance that suppresses the function of a CSABP may be used preferably as a component that inhibits a CSABP, in production of the composition or the treatment method of the present invention.
In yet another embodiment of the method of the present invention, an increase/decrease in the abundance of a CSABP due to the co-existence of a test substance may be used as an indicator for screening, and this enables a component that controls the abundance of a CSABP to be identified. A specific method for measuring an increase/decrease in the abundance of a CSABP is as described above.

Therefore, the present invention also relates to a screening kit for a component that detects and/or controls a CSABP, the kit including a CSABP or a fragment thereof. This kit contains at least a CSABP or a fragment thereof charged in any container. Examples of the container include an ampoule, a vial, and a bottle that are made of a material such as glass or plastic, and they are preferably hermetically sealed. The CSABP or a fragment thereof may be present in the form of crystals, in a freeze-dried state, or in an appropriate medium such as distilled water, physiological saline, or PBS. When the kit contains a CSABP or a fragment thereof in the form of crystals or a freeze-dried state, this kit may further contain an appropriate solvent or a reconstitution liquid (e.g. distilled water, physiological saline, PBS, etc.). The kit of the present invention may also contain, in addition to the above, various reagents used for measurement of binding properties of a test substance to a CSABP or a fragment thereof or for evaluation of a function of a CSABP such as, for example, a known CSABP-binding substance (CsA, NS5B, CyPB, anti-CSABP antibody, etc.) labeled with a radioisotope, an enzyme, a fluorochrome, etc., a probe for detection of a CSABP nucleic acid, a primer for amplification of a CSABP nucleic acid, an HCV subgenomic replicon system, a probe for detection of an HCV nucleic acid, a primer for amplification of an HCV nucleic acid, an anti-HCV antibody, etc. The above-mentioned kit may further contain various media including a description or instructions on how to use this kit such as, for example, written instructions for use, a CD, or a DVD.

The present invention also relates to a composition for controlling the growth of an RNA virus, the composition containing a component that controls the abundance and/or the function of a CSABP, and use of a component that controls the abundance and/or the function of a CSABP in the production of a composition for controlling the growth of an RNA virus.
The RNA virus that is a target of the present composition is any known virus, and examples thereof include, but are not limited to, allexivirus genus, arenaviridae, arteriviridae, astroviridae, avsunviroidae, barnaviridae, benyvirus genus, birnaviridae, bornavirus genus, bromoviridae, bunyaviridae, caliciviridae, capillovirus genus, carlavirus genus, closteroviridae, comoviridae, coronaviridae, cricket paralysis virus genus, cystoviridae, deltavirus genus, enamovirus genus, filoviridae, flaviviridae, foveavirus genus, furovirus genus, hepatitis E-like virus genus, hordeivirus genus, hypoviridae, idaeovirus genus, leviviridae, luteoviridae, marafivirus genus, metaviridae, narnaviridae, nodaviridae, ophiovirus genus, orthomyxoviridae, ourmiavirus genus, paramyxoviridae, partitiviridae, pecluvirus genus, picornaviridae, pomovirus genus, pospiviroidae, potexvirus genus, potyviridae, pseudoviridae, reoviridae, retroviridae, rhabdoviridae, sequiviridae, sobemovirus genus, tenuivirus genus, tetraviridae, tobamovirus genus, tobravirus genus, togaviridae, tombusviridae, totiviridae, trichovirus genus, tymovirus genus, umbravirus genus, varicosavirus genus, and vitivirus genus viruses.

A positive strand RNA virus is preferable for the above-mentioned composition, and examples thereof include, but are not limited to, allexivirus genus, arteriviridae, astroviridae, barnaviridae, benyvirus genus, bromoviridae, caliciviridae, capillovirus genus, carlavirus genus, closteroviridae, comoviridae, coronaviridae, cricket paralysis virus genus, cystoviridae, deltavirus genus, enamovirus genus, flaviviridae, foveavirus genus, furovirus genus, hepatitis E-like virus genus, hordeivirus genus, idaeovirus genus, leviviridae, luteoviridae, marafivirus genus, metaviridae, nodaviridae, orthomyxoviridae, ourmiavirus genus, pecluvirus genus, picornaviridae, pomovirus genus, potexvirus genus, potyviridae, retroviridae, sequiviridae, sobemovirus genus, tetraviridae, tobamovirus genus, tobravirus genus, togaviridae, tombusviridae, trichovirus genus, tymovirus genus, umbravirus genus, and vitivirus genus viruses.

Furthermore, flaviviridae viruses are more preferable in the above-mentioned composition, and examples thereof include, but are not limited to, a virus of the flavivirus genus such as yellow fever virus, Japanese encephalitis virus, dengue fever virus, or West Nile virus, a virus of the pestivirus genus such as bovine viral diarrhea virus 1 or 2, hog cholera virus, or border disease virus, a virus of the hepacivirus genus such as HCV, particularly preferred examples include a virus of the pestivirus genus such as bovine viral diarrhea virus 1 or 2, hog cholera virus, or border disease virus and a virus of the hepacivirus genus such as HCV, and most preferred examples include a virus of the hepacivirus genus such as HCV.
The above-mentioned composition may be used in cells in a living organism and also may be used in cells cultured outside a living organism. Therefore, the present composition may be used in various tests and experiments including a step of controlling the growth of the above-mentioned RNA viruses.

The present invention also relates to a method for controlling the growth of an RNA virus, the method including a step of delivering a component that controls the abundance and/or the function of a CSABP to the interior of RNA virus-infected cells. Delivery of the above-mentioned component may utilize any known method such as, for example, a method involving infusion or addition of the above-mentioned component to tissue or a medium containing infected cells, a calcium phosphate method, a lipofection method, a sonophoresis method, an electroporation method, a particle gun method, a method using a virus vector such as an adenovirus vector or a retrovirus vector, or a microinjection method. Furthermore, in order to promote delivery of the above-mentioned active component to the interior of cells, this component may be contained in an intracellular delivery carrier such as liposome, in particular a cationic liposome, may be bonded to said carrier, or may be bonded to a substance that promotes intracellular delivery such as, for example, a TAT peptide.

The present invention also relates to a composition for detecting and/or inhibiting CsA, a derivative thereof, NS5B, or CyPB, the composition containing a CSABP or a functional variant thereof, and use of CSABP or a functional variant thereof in production of a composition for detecting and/or inhibiting CsA, a derivative thereof, NS5B, or CyPB.
The CSABP functional variant in the above-mentioned composition typically means a variant having binding properties to at least one of CsA, a derivative thereof, NS5B, and CyPB. A preferred variant is one that has the above-mentioned binding properties but does not have other CSABP functions such as, for example, ATPase activity or an HCV replication promotion function, or does have them but only at a very low level. In the composition for detection, the CSABP or functional variant thereof may have attached thereto various labels for convenience of detection. The label may be any that is known and may be selected from, for example, any radioisotopes, magnetic substances, substances that bind to a CSABP or a functional variant thereof (e.g. an antibody), biotin, fluorescent substances, fluorophores, chemiluminescent substances, and enzymes.
The above-mentioned composition for detection may be used for an investigation into the biodistribution, intracellular distribution, abundance, etc. of CsA, a derivative thereof, NS5B, or CyPB, whereas the composition for inhibition may be used in the treatment of adverse symptoms due to administration of an excess amount of CsA or a derivative thereof, treatment of a disease in which NS5B or CyPB is involved such as, for example treatment of an RNA virus infection, etc.

Furthermore, the present invention relates to a method for detecting and/or inhibiting CsA, a derivative thereof, NS5B, or CyPB, the method including a step of administering an effective dose of the above-mentioned composition to a subject. In the present method, administration of the above-mentioned composition to a subject may be carried out suitably by, for example, various routes including both oral and parenteral routes, and examples thereof include, but are not limited to, oral, intravenous, intramuscular, subcutaneous, local, rectal, intraarterial, intraportal, intraventricular, transmucosal, percutaneous, nasal, intraperitoneal, intrapulmonary, and intrauterine routes. Furthermore, it is possible for a physician or a veterinarian to appropriately modify the above-mentioned administration method and administer the agent of the present invention to a subject. The effective dose referred to here is, when the object is to detect CsA, a derivative thereof, NS5B, or CyPB, an amount at which a label contained in the above-mentioned composition can be detected by an appropriate detection instrument, when the object is to treat adverse symptoms due to administration of an excess amount of CsA or a derivative thereof, an amount that alleviates the symptoms or suppresses progression, preferably an amount that eliminates the symptoms, and when the object is to treat a disease in which NS5B or CyPB is involved, an amount that reduces onset of the disease, alleviates the symptoms, or prevents progression, and preferably an amount that prevents onset of the disease or cures the disease. Furthermore, an amount that does not cause an adverse effect that exceeds the benefit of administration is preferable. Such an amount may be appropriately determined by an in vitro test using cultured cells, etc. or a test using a model animal such as a mouse, a rat, a dog, or a pig.

In the above-mentioned method, a specific dose of the composition to be administered can be determined while taking into consideration various conditions of a subject requiring treatment such as, for example, detection sensitivity of the detection instrument, type of label, severity of symptoms, general health state of the subject, age, weight, gender of the subject, diet, the timing and frequency of administration, a pharmaceutical used in combination, response to the treatment, and compliance with the treatment; it might be different from a general effective dose, but even in such a case these methods are still included in the scope of the present invention.
The frequency of administration varies according to the properties of the composition used and the conditions of the subject as described above but may be, for example, a plurality of times per day (that is, 2, 3, 4, 5, or more times per day), once a day, every few days (that is, every 2, 3, 4, 5, 6, or 7 days, etc.), once a week, once every few weeks (that is, once every 2, 3, or 4 weeks, etc.).

Furthermore, the present invention also relates to a method for detecting CsA, a derivative thereof, NS5B, or CyPB, the method including a step of contacting the composition for detection with a sample collected from a subject.
Detection may employ any known method. Examples of such a method include, but are not limited to, an immunoprecipitation method, EIA, ELISA, IRA, IRMA, western blotting method, immunohistochemical method, and immunocytochemical method, which utilize an anti-CSABP antibody, and an enzyme chromogenic method, fluorescence detection method, radioactivity measurement method, autoradiographic method, flow cytometric method, tissue staining method, and cell staining method, which utilize the above-mentioned composition containing a label.
A sample used for the above-mentioned detection includes, for example, blood, a mucosal scraping, various cells or organs collected by surgery, a biopsy, etc., cultured cells, a cell culture, etc. The sample may be subjected to a detection operation as it is, but if, for example, cells are disrupted by sonication, etc. and a cell extract is prepared from the centrifugal supernatant, the detection operation becomes easy.

The present invention also relates to a composition for controlling an RNA-metabolizing system, the composition containing a component that controls the abundance and/or the function of a CSABP, and use of a component that controls the abundance and/or the function of a CSABP in the production of a composition for controlling an RNA-metabolizing system. Since CSABP has a DExDc domain (191st to 378th amino acids), it is assumed to have RNA helicase activity. RNA helicase can dissociate RNA/RNA or RNA/DNA double strands into single strands, and is involved in RNA-metabolizing systems such as mRNA maturation, ribosome binding, and translation. Since it is thought that CSABP is also involved in RNA-metabolizing systems in a similar manner, by controlling the abundance or the function of CSABP, it can be expected that the RNA-metabolizing systems will be controllable. In Example 5 of the present description it is shown that the amount of HCV RNA is reduced by lowering the amount of expression of CSABP, and in Example 6 it is shown that the amount of HCV RNA is increased by overexpressing CSABP; this suggests that the RNA-metabolizing system can be controlled by controlling CSABP.

Therefore, the above-mentioned composition can be used for treatment of an RNA-metabolizing system-related disease such as a disease resulting from an abnormality of the RNA-metabolizing system in which a CSABP is involved or a disease that is improved by controlling the metabolizing system, and examples thereof include infection by a pathogen that utilizes RNA for replication such as an RNA virus, a tumor, a testicular proliferative disease, a disease resulting from an abnormality in a bioactive substance produced in the testicles, for example, a sex hormone such as testosterone (a prostate disease such as prostate cancer or prostatic hyperplasia, male climacteric disorder, testicular hypofunction) and, moreover, it can also be used in a test or an experiment, etc. requiring control of an RNA-metabolizing system.
Furthermore, the present invention relates to a method for treating an RNA-metabolizing system-related disease, the method including a step of administering an effective dose of the above-mentioned composition to a subject. Other details of the present method are in accordance with the method for treating a CSABP-related disease described above. Furthermore, the present invention relates to a method for controlling an RNA-metabolizing system, the method including a step of adding the above-mentioned composition to a sample that requires control of the RNA-metabolizing system. The above-mentioned composition is preferably added at an effective dose. The effective dose referred to here means an effective amount at which the above-mentioned component promotes, maintains, or suppresses the RNA-metabolizing system, and such an amount can be appropriately set by a person skilled in the art. Examples of samples in the present method include cultured cells, cultured tissue, extracted cells, and extracted tissue.

The present invention also relates to a CSABP expression site-specific drug delivery system containing a substance that binds to a CSABP, and use of a substance that binds to a CSABP in the production of a CSABP expression site-specific drug delivery system. Since CSABP is localized at a specific site (e.g. endoplasmic reticulum, etc.) of a specific cell (e.g. testicular or tumor cells, etc.) (see e.g. Example 7 (2) in the present description), a substance that binds to a CSABP may be utilized as a targeted molecule for a CSABP expression site. Examples of such a substance include an anti-CSABP antibody, CsA, a derivative thereof, NS5B, CyPB, and a CSABP-binding variant thereof. The CSABP-binding variant of NS5B or CyPB is preferably a variant that binds to a CSABP but lacks other physiological activities. Examples of such a variant include an NS5B variant that contains a CSABP-binding site but does not contain a CyPB binding site (in the case of HCV, the 521st to 591st amino acids) and an NS5B variant that contains a CSABP-binding site but does not contain an RNA polymerase region (in the case of HCV, the 116th to 398th amino acids). The substance that binds to a CSABP is bound to a drug that is delivered by any known method. For example, when both the substance that binds to a CSABP and the drug that is delivered are proteins, nucleic acids coding for the two may be bound by genetic engineering to form a fusion protein. In order to make uptake into cells easy, a complex of a CSABP-binding substance and a drug may be incorporated into liposome, and preferably cationic liposome, or form an emulsion therewith.

The drug that is delivered includes any known drug that exhibits its action at a CSABP expression site or for which the action is enhanced at the site. For example, since various enzymes that are involved in synthesis of a sex hormone such as testosterone, such as for example 17α-hydroxylase, 17,20 lyase, 3β-hydroxysteroid dehydrogenase, and 17β-hydroxysteroid dehydrogenase, are present in the endoplasmic reticulum of the testicle, as one example of the above-mentioned drug, there can be cited these enzymes themselves, drugs that promote or suppress the activity of these enzymes such as, for example, trilostane, which is a 3β-hydroxysteroid dehydrogenase inhibitor, and a 17β-hydroxysteroid dehydrogenase inhibitor described in JP-A-10-259182, etc. Furthermore, when the intention is to deliver to tumor cells, various drugs for the purpose of treating a tumor may be delivered. Examples of such drugs include, but are not limited to, those described above. Further examples of drugs to be delivered include various labeling substances such as, for example, any radioisotope, magnetic substance, fluorescent substance, fluorophore, chemiluminescent substance, and enzyme.
The present invention also relates to a method of CSABP expression site-specific delivery of a drug employing the above-mentioned CSABP expression site-specific drug delivery system. This method may include a step of administering an effective dose of the above-mentioned drug delivery system to a subject, and in this case other details are in accordance with the method for treating a CSABP-related disease described above. The present method may also include a step of adding in vitro the above-mentioned drug delivery system to a sample such as cultured cells, cultured tissue, extracted cells, or extracted tissue.

The present invention also relates to a method for screening a substance that controls CSABP gene expression, the method including 1) a step of making a cell having a nucleic acid molecule containing a CSABP promoter and a reporter sequence operably linked thereto coexist with a test substance, and 2) a step of detecting an expression product of the reporter sequence.
The CSABP promoter referred to here is a portion of a gene that is positioned on the upstream side (5' side) of the coding region of CSABP gene and controls expression of the CSABP gene, and typically has a transcription factor binding sequence. The promoter region containing a human CSABP promoter is present in a portion containing bases -463 to -1 (SEQ ID No: 37), preferably bases -463 to -44 (SEQ ID No: 30), and particularly at least one of transcription factor binding sites (bases -220 to -213: (CREB binding site: TGACGTCA), bases -188 to -179 (GATA binding site: CGTGATATCA), and bases -150 to -140 (AP-1 binding motif: GGTGACTGAGG)). The promoter region of a non-human CSABP may be appropriately determined by, for example as in Example 8, transfecting a cell, preferably an allogenic cell, with a nucleic acid molecule containing various lengths of a cDNA fragment containing a sequence upstream of a coding region of CSABP and a reporter sequence operably linked thereto, and by detecting expression of the reporter sequence.

As the reporter sequence, any base sequence for which an expression product therefrom is detectable may be used. For convenience of detection, for example, alkaline phosphatase (ALP), chloramphenicol acetyl transferase (CAT), β galactosidase (βgal), β glucuronidase (βglu), β lactamase, green fluorescent protein (GFP), various luciferases (e.g. firefly luciferase, sea pansy luciferase, railroad worm luciferase), etc. genes may be used, but they are not limited thereto. That is, when the expression product is a protein, it can be detected by use of a specific antibody, and when the expression product is an mRNA, it can be detected by use of a specific nucleic acid probe. Therefore, as the reporter sequence a CSABP gene may also be employed.
Cells used in the present method are not particularly limited but are preferably animal cells, more preferably mammal cells, and most preferably cells allogenic with a CSABP of interest such as, for example, human cells. Cells may originally contain the above-mentioned nucleic acid molecule or may be artificially introduced with the above-mentioned nucleic acid molecule. Introduction of a nucleic acid molecule may be carried out by any known method, and examples thereof include, but are not limited to, a calcium phosphate method, a lipofection method, a sonophoresis method, an electroporation method, a particle gun method, a method utilizing a virus vector such as an adenovirus vector or a retrovirus vector, and a microinjection method. Therefore, one embodiment of the present method may include a step of transfecting a cell with the above-mentioned nucleic acid molecule.

The test substance in the present method is not particularly limited, and includes a polymer such as a protein or various low molecular weight compounds. The test substance may be a known substance or may be a novel substance that is synthesized by any known method such as recombinatorial chemistry. Examples of the test substance include, but are not limited to, substances involved in control of transcription, in particular substances related to transcription factor CREB, GATA, or AP-1, for example, PKA, cAMP, adenylate cyclase, p90^{RSK}, ERK, p38, MAPKAPK-2, MSK-1, CaMK, calcium ion, Akt/PKB, JNK, FRK, RSK, casein kinase II, GSK-3β, and substances related thereto. Furthermore, it has now become clear that a CSABP promoter region containing a CREB-binding motif forms a CpG island and this enables methylation to be carried out, and since it is thought that the degree of methylation of the promoter influences the transcription activity (see e.g. Bernstein BE et al., Cell. 2007; 128 (4): 669-81), a substance that controls methylation, for example, an anti-methylating agent, etc. may be cited as an example of the test substance. It is also possible to use as the test substance of the present method a substance identified by a method for screening a substance that controls expression of a CSABP gene, the method including a step of recovering a substance that binds to the CSABP promoter, which is described later. Therefore, the present method may further include a step of recovering a substance that binds to the CSABP promoter prior to step 1). Furthermore, as described later, the step of recovering a CSABP promoter-binding substance may further include a step of purifying the recovered CSABP promoter-binding substance and/or a step of identifying the recovered CSABP promoter-binding substance (or the purified CSABP promoter-binding substance).

Co-existence with a test substance may be appropriately achieved by any known method and includes, but is not limited to, for example, addition of a test substance to a culture system containing cells, expression of said substance in cells by introduction with a nucleic acid coding for a test substance, etc.
An expression product of the reporter sequence may be in various forms including, but not limited to, for example, a transcription product, a splice product, a translation product, and a post-translational modification product, and detection may be carried out by a detection method suitable for each form. That is, when the expression product is a nucleic acid, for example, an mRNA, examples of the method include, but are not limited to, various hybridization methods, a northern blot method, various PCR methods, etc., utilizing a nucleic acid that specifically hybridizes with said nucleic acid, and when the expression product is a polypeptide, there are an immunoprecipitation method, EIA, ELISA, IRA, IRMA, western blotting method, immunohistochemical method, immunocytochemical method, enzyme chromogenic method, fluorescent detection method, radioactivity measurement method, autoradiographic method, flow cytometric method, tissue staining method, cell staining method, etc., which utilize an antibody that specifically binds to said polypeptide.

In the present method, a substance that increases the amount of expression of a reporter sequence is determined to be a substance that enhances expression of a CSABP gene, and a substance that reduces the amount of expression of a reporter sequence is determined as a substance that suppresses expression of a CSABP gene. Increase or decrease of the expression level here typically means an increase or decrease when compared with the expression level in the absence of a test substance. A CSABP expression control substance identified by the present method is also included in the scope of the present invention.
In the present method, in order to guarantee the accuracy of detection and the feasibility of comparison, a positive control and/or a negative control may be provided as appropriate. Specific provision of a positive control and/or a negative control is within the scope of the ability of a person skilled in the art.

The present invention also relates to a method for screening a substance that controls expression of a CSABP gene, the method including a step of recovering a substance that binds to a CSABP promoter.
As methods for recovering a substance that binds to a CSABP promoter in the present method there can be cited, for example, a method in which a biotinylated CSABP promoter and a test substance such as for example a cell homogenate or a cell lysate are contacted with avidin or streptavidin fixed to a solid phase (e.g. beads, plate, chip, column, etc.) to thus pull-down the test substance, a method in which after a test substance is contacted with a CSABP promoter bound to a solid phase, the solid phase is washed to thus recover a complex of the promoter and a substance that is bound thereto, etc. Instead of biotin or avidin, another known binding carrier, preferably a binding carrier that can be applied to a nucleic acid such as, for example, digoxigenin and anti-digoxigenin antibody, may be used. Furthermore, contacting a biotinylated CSABP promoter and a test substance with a solid phased avidin, etc. may be carried out by simultaneously mixing the three, or it may be achieved by mixing a biotinylated CSABP promoter and either one of the other two in advance and then adding the remaining one.
A substance that is bound to a CSABP promoter may be identified by, for example, MS (mass spectrometry), etc. Before carrying out MS, a recovered test substance may be subjected to purification by one-dimensional or two-dimensional gel electrophoresis or a chromatographic method such as liquid chromatography. Therefore, the present method may further include a step of purifying a recovered CSABP promoter-binding substance and/or a step of identifying a recovered CSABP promoter-binding substance (or a purified CSABP promoter-binding substance).

The present invention also relates to a CSABP promoter. Said promoter is a portion of a gene for controlling expression of a CSABP gene, positioned on the upstream (5' side) of the coding region of CSABP gene, and typically has a transcription factor-binding sequence. A human CSABP promoter is selected from the group consisting of i) a nucleic acid molecule that contains the base sequence represented by SEQ ID No: 37 or a portion thereof, ii) a nucleic acid molecule that has one or more mutations, preferably one or a few, in the base sequence of nucleic acid molecule i), but that still has equivalent functions to said nucleic acid molecule, iii) a nucleic acid molecule that hybridizes under stringent conditions with a complementary strand or a fragment thereof of nucleic acid molecule i) or ii), and that has equivalent functions to said nucleic acid molecule, and iv) a nucleic acid molecule that has at least 60% homology to nucleic acid molecule i) or ii), preferably at least 70%, more preferably at least 80%, yet more preferably at least 90%, and particularly preferably at least 95%, and that has equivalent functions to said nucleic acid molecule.

Therefore, the human CSABP promoter contains a base sequence comprising bases -463 to -1 (SEQ ID No: 37) or a portion thereof, preferably a base sequence comprising bases - 463 to -44 (SEQ ID No: 30) or a portion thereof, and particularly a CSABP gene portion containing at least one of transcription factor binding sites (bases -220 to -213: (CREB binding site: TGACGTCA), bases -188 to -179 (GATA binding site: CGTGATATCA), and bases -150 to -140 (AP-1-binding motif: GGTGACTGAGG)).
The portion of the base sequence here is preferably functional. Whether or not a given gene portion is functional may be determined as appropriate by, for example, operably linking this portion and the above-mentioned reporter sequence, transfecting a predetermined cell therewith, and detecting expression of the reporter sequence (see e.g. Example 8).
Furthermore, the non-human CSABP promoter may be determined as appropriate by, for example as in Example 8, transfecting cells, preferably allogenic cells, with a nucleic acid molecule containing various lengths of cDNA fragment containing the upstream sequence of the coding region of CSABP, and a reporter sequence operably linked thereto, and detecting expression of the reporter sequence, and a non-human CSABP promoter thus obtained is also included in the present invention.
The CSABP promoter of the present invention may be used in the above-mentioned method for screening a substance that controls expression of a CSABP gene, a method for determining the onset or disease stage of a CSABP-related disease described below, etc.

The present invention also relates to a vector and a cell having the CSABP promoter. In said vector and cell, the CSABP promoter may be operably linked to the reporter sequence. With regard to the CSABP promoter, the reporter sequence, the vector, and the cells, any of those described with respect to the method for screening a substance that controls expression of a CSABP gene may be used. The cells of the present invention may be ones that have not been subjected to artificial modification but are typically ones that have been subjected to artificial modification such as gene introduction. The vector and cells may be used in the above-mentioned method for screening a substance that controls expression of a CSABP gene, etc.

The present invention also relates to a method for determining the onset or disease stage of a CSABP-related disease, the method including a step of detecting methylation in a CSABP gene, for example, in its coding region or noncoding region such as the promoter region.
As hereinbefore described, it has been clarified that the CSABP promoter region containing a CREB-binding motif forms a CpG island, this enabling methylation to be carried out, and it has been found that by administration of an anti-methylating agent to cells, expression of CSABP can be enhanced (see Example 9). As described in Example 2 below, while taking into consideration that expression of CSABP is very low in normal tissue and is markedly high in cancer cells, it is assumed that there is a strong link between removal of methylation in the CSABP gene and malignant transformation of cells. Therefore, by detecting methylation in the CSABP gene, it becomes possible to determine the onset or disease stage of a CSABP-related disease such as, for example, a neoplastic disease.

The CSABP promoter region and the CSABP-related disease in the present method are as described above. In the present method, methylation may be detected by any known method including, but not limited to, for example, methylation specific PCR (MS-PCR), combined bisulfite restriction assay (COBRA), MS-SNuPE, bisulfite-SSCP, a DMH (differential methylation hybridization) method, a MethyLight method, or a Pyrosequencing method.
In the present method, for example, blood, saliva, oral mucosa, blister contents, a skin fragment containing a blister, feces, urine, various cells, body fluids, organs, etc. collected by surgery, a biopsy, etc. may be used as samples. These samples may be subjected to detection as they are, but preferably DNA is extracted/purified in advance. An extraction method for DNA may be selected from any method known in the present technical field, for example, a method such as a proteinase K/phenol extraction method, a proteinase K/phenol/chloroform extraction method, an alkaline lysis method, or a boiling method may be used as appropriate.
In accordance with the present method, for example, with regard to a CSABP-related disease characterized by expression of CSABP, for example, when the degree of methylation of a test subject-derived sample is lower than that of a healthy subject-derived sample, it is determined that the CSABP-related disease in the test subject is in onset; when the degree of methylation of a test subject-derived sample at a certain point in time is lower than that of the test subject-derived sample at a point in time prior thereto, it is determined that the CSABP-related disease in the test subject is in progression; and when the degree of methylation of a test subject-derived sample at a certain point in time is higher than that of the test subject-derived sample at a point in time prior thereto, it is determined that the CSABP-related disease in the test subject is in remission.

### Examples

The present invention is explained below more specifically by reference to Examples, but the present invention should not be construed as being limited to these Examples.

### Example 1 Cloning of CSABP

CsA-binding human intracellular protein was cloned using a phage display method. First, CsA was photochemically covalently bonded to PEG resin beads (CsA beads). 0.5 mg of CsA beads was added to 1 × 10¹¹ pfu of a random 12-peptide phage library (prepared using T7Select^{®} 10-3 OrientExpress cDNA Cloning System, Oligo (dT) (Novagen)), and incubation was carried out at 4°C overnight. The beads were washed with 1 mL of 1×TBST (50 mM Tris, pH 8.0, 150 mM NaCl, 0.1% Tween20) a total of 10 times, 100 µL of infectable E. coli was then added thereto, and phage was collected directly from the beads. 0.5 mg of beads were added again to 1 × 10¹¹ pfu of the phage thus collected, and the series of operations was repeated a total of 4 times. 5 independent phages were amplified individually from the 4^{th} collected phage, about 1 × 10¹⁰ pfu of phage was incubated with 0.5 mg of beads at 4°C overnight, and 1% SDS was added thereto to lyse the phage. The proportion of the phage lysis was calculated based on beads to which CsA was not bound.

Phage clone No. 13, for which the proportion lysis was the highest, was cloned. This phage displayed a peptide having an LVFGTLLGQLRA amino acid sequence. When a gene coding for this peptide was queried in the NCBI database (http://www.ncbi.nlm.nih.gov/), a human-derived protein having homology with this sequence was found (gene ID: NM_022828). This protein consisted of a total length of 1430 amino acids and contained the LLGQLRA sequence halfway along. This protein was a CSABP.

### Example 2 Analysis of CSABP expression

CSABP expression conditions in various tissues and cell lines were analyzed by a northern blot method and an RT-PCR method.

### (1) Northern blot analysis

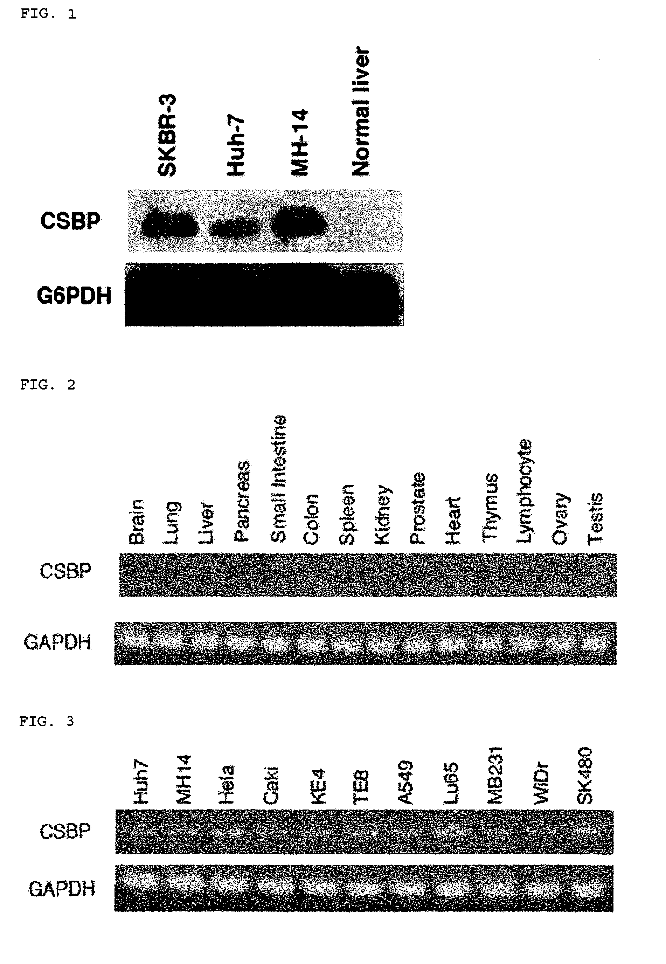

Northern blotting was carried out based on a standard method. The total RNAs used were from normal human liver tissue (Human Liver Total RNA, Clontech), an SKBR-3 human breast cancer cell line, an Huh-7 human liver cancer cell line, and an MH14 HCV genome replicon cell line, and extraction of total RNA from each cell line was carried out using an RNeasy kit (Invitrogen). 20 µg of an RNA sample was subjected to electrophoresis using a formaldehyde-denatured gel. After the electrophoresis was completed, the gel was transferred to a nylon membrane (Hybond-N +) overnight, crosslinked by UV crosslinking, and dried. Hybridization was carried out by a random primer method (Rediprime II, GE healthcare) using CSABP gene labeled with ³²P as a probe (5'-acgaagtgatgggtgggagcgagcc-3' (SEQ ID No: 3) and 5'-cccataactggagcaactgttcacc-3' (SEQ ID No: 4)), and CSABP RNA in each sample that bound to the CSABP probe was detected using an X-ray film (BioMax (Kodak), FUJI MEDICAL X-RAY FILM (FUJIFILM)) (FIG. 1). From the results, a single band around base 4300 was confirmed for all three types of cancer cells investigated. However, such a band could not be observed for normal liver cells.

### (2) Analysis of expression by RT-PCR

First strand cDNA libraries (Clontech) from normal tissue in 14 types of organs and total RNA recovered from 11 types of cancer cells were used as templates. Recovery of total RNA from the cancer cells and a reverse transcription reaction of the total RNA obtained were carried out by standard methods using an RNeasy kit (Qiagen) and a Transcriptor First Strand cDNA Synthesis Kit (Roche) respectively. With regard to primers, CSABP 791 to 810 (5'-gacgggaaaggattggtcaa-3', SEQ ID No: 5) was used as a forward primer, CSABP 2723 to 2742 (5'-cccatcacttcgtgcttttt-3', SEQ ID No: 6) was used as a reverse primer, and a PCR reaction was carried out using a Gotaq^{®} PCR system (Promega) at 95°C, 5 minutes × 1 cycle, 95°C, 0.5 minutes, 55°C, 0.5 minutes, 72°C, 2.5 minutes × 35 cycles, 72°C, 7 minutes × 1 cycle. The results are shown in FIGS. 2 and 3.
Expression of CSABP was very low in almost all normal tissues except testicular (FIG. 2), but was prominent for all of the cancer cells investigated (FIG. 3). This suggests that CSABP can be used as a testicular marker or a cancer marker.

### Example 3 Synthesis of CSABP recombinant protein

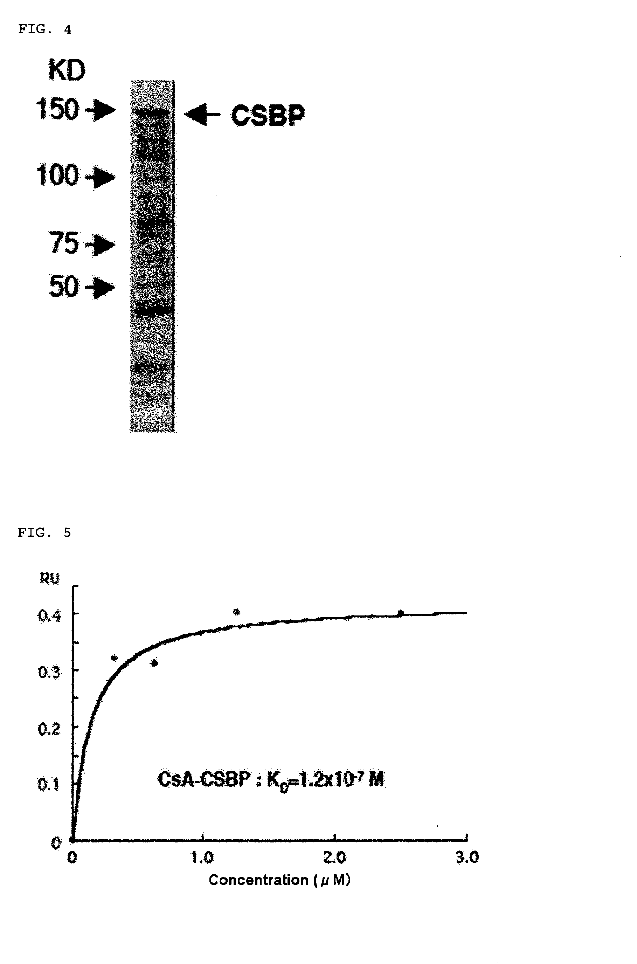

CSABP gene was amplified by PCR using a primer with EcoRI and XhoI recognition sequences added (forward primer: 5'-CCGAATTCATGTCCAGGCCGAGCAGCGTCTCC-3' (SEQ ID No: 7), reverse primer: 5'-CCCTCGAGTCAATCAGTTGTGTTTTTTTCTCCC-3' (SEQ ID No: 8) (the underlining denotes restriction enzyme recognition sequences)) with a cDNA library of human breast cancer cell line SKBR-3 as a template, and cloned into pET vector (Invitrogen). pET-CSABP-FL and pET-CSABP-C (FL = full length, C = C terminal region (CSABP ORF: 193rd to 1430th amino acids)) were introduced into E. coli (BL21 (DE3)), and cultured in the presence of 0.1 mM IPTG at 20°C overnight to thus express protein. E. coli that had induced expression of CSABP was recovered, a soluble fraction was purified using HisTrap (Amersham), and following this the purified protein was checked for molecular weight by SDS-PAGE (Coomassie staining) (FIG. 4). A band of the purified protein was observed at a molecular weight of around 150 kDa, this coinciding with information given in the NCBI database. It became therefore clear that a sufficient amount of recombinant CSABP protein can be prepared by the above-mentioned method.

### Example 4 Evaluation of functions of CSABP

The ability of CSABP to bind to CsA and ATPase activity were evaluated as follows.

### (1) Molecular association with CsA

Molecular association between recombinant full length CSABP protein (CSABP-FL) and CsA was evaluated by an SPR (surface plasmon resonance) method. SPR measurement employed Biacore 3000 (Biacore). A purified recombinant CSABP protein was immobilized on Biacore chip CM5 by an amine coupling method (amount immobilized about 10000). CsA dissolved in PBS-8% DMSO was used as an analyte. The RU value of CSABP was measured for various CsA concentrations, and a K_{D} value was calculated using an application supplied with the Biacore. From the results, the dissociation constant between CsA and CSABP was calculated to be K_{D} = 1.2 × 10⁻⁷ M, and it became clear that the two substances bind specifically to each other (FIG. 5).

### (2) Measurement of ATPase activity

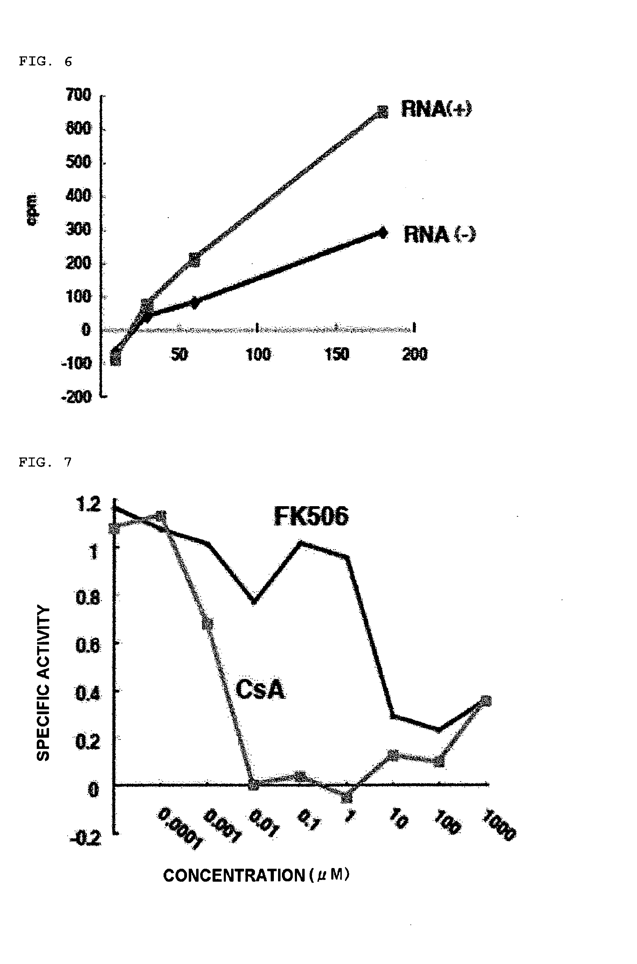

Measurement of ATPase activity employed the above-mentioned method by Okanami et al. That is, purified CSABP-C was concentrated by 40 times and subjected to exchange with PBS buffer (137 mM NaCl, 10 mM phosphoric acid, 2.7 mM KC1, pH 8.0). Purified CSABP-C (500 ng) was added to 50 µL of helicase buffer (30 mM Tris-HCl (pH 7.5), 8 mM MgCl₂, 100 mM NaCl, 15 mM DTT), and a reaction was carried out at 30°C in the presence of 1 µL of 30 nM [γ-³²P] ATP (1 Ci/mmol) and 100 ng of total RNA (liver-derived). The reaction was halted by addition of an excess amount of phosphoric acid, ATP was made to adsorb on active carbon, and the amount of ³²P released by decomposition was then measured by a scintillation counter to thus give ATPase activity. Furthermore, for comparison, measurement was carried out in the same manner under conditions in which RNA was absent. The results are shown in FIG. 6. It can be seen from the figure that CSABP has RNA-dependent ATPase activity.
In order to examine change in ATPase activity of CSABP in the presence of CsA, the measurement was carried out in the same manner as above in the presence of various concentrations of CsA or FK506, and ATPase activity was calculated (FIG. 7). From this, it became clear that the ATPase activity of CSABP is subject to functional inhibition by binding of CsA. The dissociation constant between FK506 and CSABP was K_{D} = 2.5 × 10⁻⁶ M.

### Example 5 Effect of suppression of expression of CSABP on HCV replication

The effect of suppressing expression of CSABP by siRNA on HCV replication was examined using MH14 cell system. It is known that MH14 cells are ones in which an HCV subgenome from which infectivity has been removed is introduced into human liver cancer cell line Huh-7 (Miyanari Y et al., J Biol Chem. 2003 Dec 12; 278 (50): 50301-8); HCV subgenome is expressed in MH14 cells, and its replication mechanism is the same as that of the wild type.

### (1) Preparation of siRNA and introduction into MH14 cells

siRNAs targeting 6 different types of regions of the CSABP gene were chemically synthesized by a standard method (see the table below). Furthermore, β-actin specific siRNA (Ambion) was used as a control (si-control).

**[Table 1]**

| siRNA ID | Sequence(sense strand only) |
|---|---|
| #127336 | 5' GGACAUUCGCAUUGAUGAGtt (SEQ ID NO: 9) |
| #127337 | 5' GGGAGCAAAUAGAUACCUAtt (SEQ ID NO: 10) |
| #127338 | 5' CCUGUAAUUUGACUCAUAAtt (SEQ ID NO: 11) |
| #30311 | 5' GGAGCUUUCAGUGACCAUAtt (SEQ ID NO: 12) |
| #30403 | 5' GGUCAAAUAAUAGUAGGAAtt (SEQ ID NO: 13) |
| #302I7 | 5' GGCCUCUCAAAAACGUGCAtt (SEQ ID NO: 14) |

Introduction of siRNA to MH14 cells was carried out as follows. MH14 cells at a concentration of 2 × 10⁵ cells/well were cultured in a 6 well plate overnight (37°C, 5% CO²), a suspension of each siRNA in a 5% siLentFect (BIORAD) solution at a final concentration of 30 nM was added to the MH14 cells, culturing was carried out for 4 hours, half of the medium was replaced with fresh medium containing 15 nM of siRNA, 18 hours thereafter the entire amount of medium was replaced with fresh medium, and culturing was carried out for a further 5 days.

### (2) Quantitative determination of gene expression

After culturing, the cells were collected, the total RNA was purified by an RNeasy kit (Qiagen), a reverse transcription reaction was carried out by a standard method, and first strand cDNA was thus prepared. Each of G6PDH, CSABP, and HCV genes was quantitatively determined by real time PCR using the cDNA thus obtained. G6PDH was used as an internal control in order to correct for the amount of RNA between cells. Quantitative determination was carried out by a real time RT-PCR method for the G6PDH and HCV genes and by a Cybergreen method for the CSABP gene using the primer and/or probe shown in the table below. Quantitative determination of G6PDH employed a double-probe method. With regard to CSABP, a primer for siRNA #30311 introduced cells is shown.

**[Table 2]**

| Gene | Forward primer | Reverse primer | Fluorescent probe |
|---|---|---|---|
| HCV | CGGGAGAGCCATAGTGG (SEQ ID NO: 15) | AGTACCACAAGGCCTTTCG (SEQ ID NO: 16) | CTGCGGAACCGGTGAGTACAC (SEQ ID NO: 17) |
| CSBP | GTGTCTGGACCCCATCCTTA (SEQ ID NO: 18) | CCCATCACTTCGTGCTTTTT (SEQ ID NO: 19) | |
| G6PDH | CTGCGTTATCCTCACCTTC (SEQ ID NO: 20) | CGGACGTCATCTGAGTTG (SEQ ID NO: 21) | 3' FITC-labeled probe: GGGGCTATTTCGATGAATTTGGGATCAT (SEQ ID NO: 22) 5' LC Red 640 probe CGGGACGTGATGCAGAACCACCTACTG (SEQ ID NO: 23) |

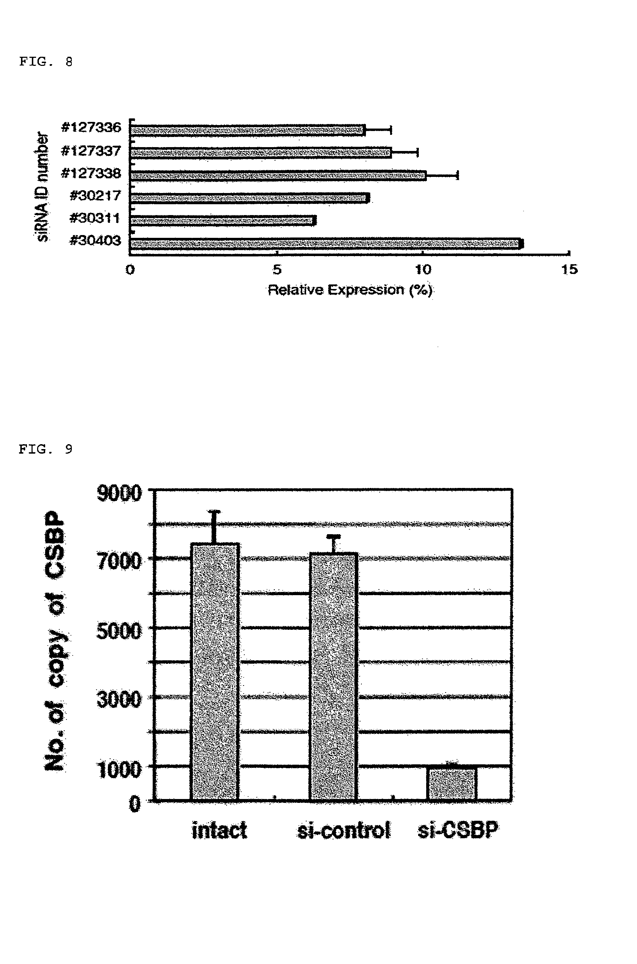

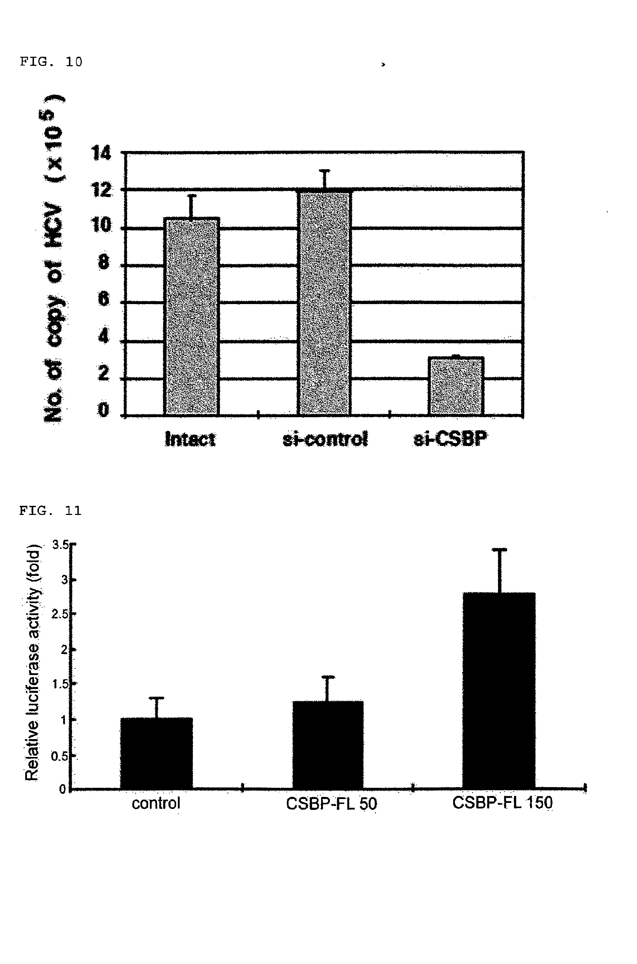

From the results, it became clear that all of the 6 types of siRNA-CSABPs (si-CSABP) targeting CSABP can suppress transcription of CSABP at the mRNA level, and among them #30311 shows the best suppression efficiency (FIG. 8). Furthermore, in accordance with introduction with si-CSABP (#30311), the amount of expression of CSABP and the amount of replication of HCV genome were remarkably reduced to 1/7 or less and 1/4 or less respectively (FIGS. 9 and 10).

### Example 6 Effect of overexpression of CSABP on HCV replication

The CSABP gene was exogenously introduced, and the resulting effect on HCV replication was examined.
First, the full length cDNA coding for CSABP protein obtained in Example 3 was introduced into the BamHI/NotI site of mammalian expression vector pcDNA3, thus preparing CSABP-pcDNA. This vector was suspended in Lipofectamin 2000 (Invitrogen) at a concentration of 50 or 150 ng/mL, and RNA having a neomycin-resistant gene replaced by a luciferase gene in HCV subgenomic replicon sequences was introduced into LMH14 cells (see Non-Patent Document 4). On the 4^{th} day after introduction, luminescence due to expression of the luciferase gene was measured, and HCV replication was quantitatively measured. The results are shown in FIG. 11. As is clear from this figure, CSABP-pcDNA enhanced replication of the HCV genome in a dose-dependent manner.

### Example 7 Analysis of mechanism of replication of HCV genome by CSABP

In order to clarify the mechanism by which CSABP is involved in replication of the HCV genome, searching for the target molecule of CSABP within the cell was carried out in vitro and in vivo.

### (1) In vitro molecular association

First, in order to prepare a CSABP-GST fusion protein, CSABP-cDNA obtained in Example 3 was inserted into the EcoRI/XhoI site of pGEX-6P-1 (GE healthcare), and introduced into E. coli Rosetta (DE3) (Novagen). Cloning of the CSABP-pGEX transformant was carried out based on a standard method, and culturing was carried out for expression of CSABP protein. After culturing was carried out for a predetermined period of time, IPTG was added thereto, expression of the CSABP-GST fusion protein was induced, and the transformant thereof was collected. Purification of CSABP-GST fusion protein was carried out in accordance with the GE Healthcare protocol, thus giving CSABP-GST fusion protein.

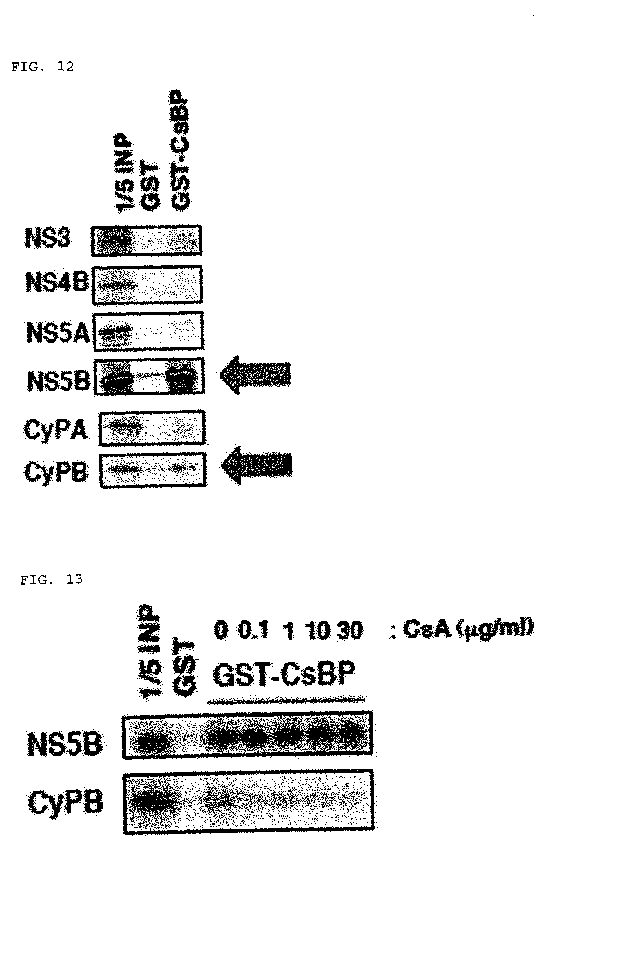

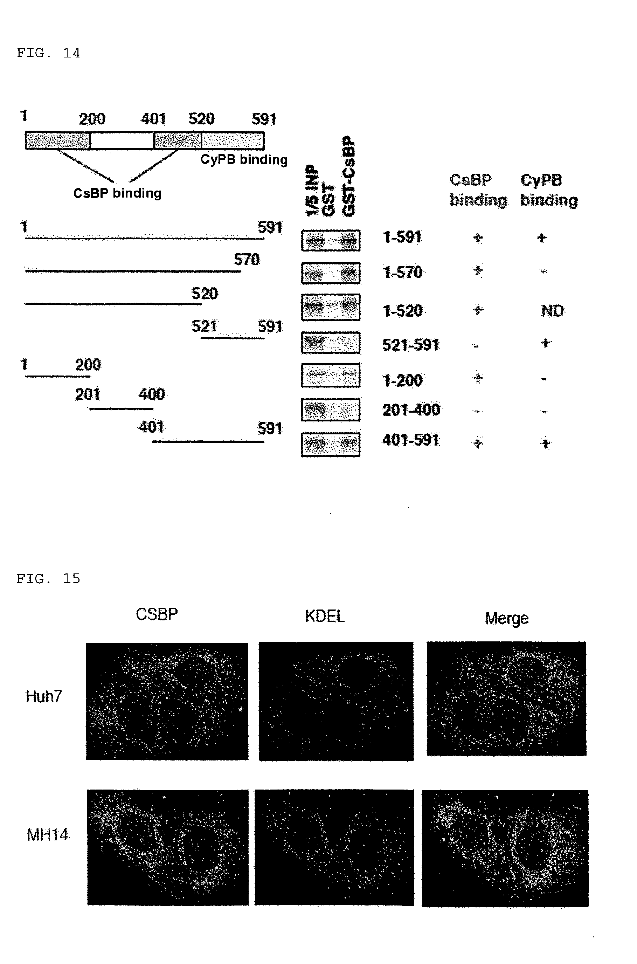

Subsequently, the CSABP-GST fusion protein was mixed with each of NS3, NS4B, NS5A, NS5B, CyPA, and CyPB proteins formed by 35S-labeled protein synthesis in vitro, and after culturing was carried out for 2 hours, recovery was carried out using a GST column. For the control, 1/5 amount of protein solution supplied to the binding experiment was used. These samples were analyzed using a BAS5000 imaging analyzer, and each of NS3, NS4B, NS5A, NS5B, CyPA, and CyPB proteins was detected. Furthermore, in another experiment, CsA was added at a concentration of 0, 0.1, 1, 10, and 30 µg/mL in the co-culture of the above-mentioned in vitro synthesis protein solution and the CSABP-GST protein, and after culturing was carried out for a predetermined period of time, a sample that had coprecipitated with GST-CSABP using glutathione Sepharose was detected in the same way. The results are given in FIGS. 12 and 13.
Since NS5B and CyPB (portion shown by arrow in FIG. 12) were detected in the sample in which, after coculturing the GST-CSABP and the in vitro synthesis protein solution, the GST-CSABP was recovered by glutathione Sepharose, it became clear that CSABP associates with NS5B and CyPB. This shows that CSABP forms a trimer with CyPB and NS5B. On the other hand, association of GST-CSABP/NS5B was maintained by addition of CsA, but CyPB dissociated from these molecules (FIG. 13).
In yet another experiment, which portion of NS5B the CSABP binds to was investigated using various NS5B fragments. From the results, it became clear that, of NS5B, CSABP binds to a portion comprising the 1st to 200th amino acids and a portion comprising the 401st to 520th amino acids (FIG. 14). This is in contrast to CyPB, which binds to the C terminal of NS5B (521st to 591st amino acids) (see Non-Patent Document 10).

### (2) In vivo molecular association

### 1) Intracellular localization of CSABP

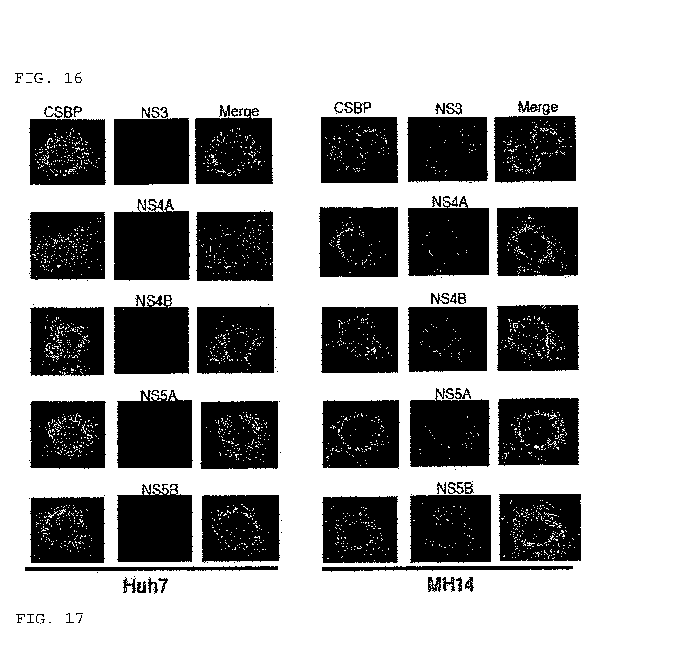

In order to examine whether or not CSABP is actually present within cells, an anti-CSABP anti-serum (polyclonal antibody) was prepared, and localization of CSABP within Huh7 cells and MH14 cells was examined by immunostaining. Furthermore, whether or not CSABP is colocalized with a molecule involved in replication of HCV was investigated using the anti-CSABP anti-serum.
First, a peptide (ILHPKRGTEDRSDQ, corresponding to the 1237th to 1250th amino acids of SEQ ID No: 1) of part of the amino acid sequence that the CSABP gene codes for was synthesized; a rabbit was immunized therewith 4 times by a standard method, after a predetermined period of time rabbit serum was collected and concentrated, a peptide column of the peptide used for immunization was prepared, and antibodies that specifically adsorbed thereon were recovered, thus giving a purified anti-CSABP anti-serum. After Huh7 cells and MH14 cells were fixed by cold acetone, they were subjected to double staining using the above-mentioned anti-CSABP antibody and an antibody to endoplasmic reticulum membrane protein KDEL present within cytoplasm. Staining employed an anti-rabbit IgG-Alexa 488-labeled secondary antibody for the CSABP antibody and anti-rabbit IgG-Alexa 594-labeled secondary antibody for the KDEL antibody (both from Molecular Probes) respectively.
Furthermore, in another experiment, double staining was carried out using, instead of the anti-KDEL antibody, an antibody to various HCV-derived proteins (NS3, NS4A, NS4B, NS5A, and NS5B), and colocalization of CSABP in the HCV replication site was also examined. Staining employed an anti-rabbit IgG-Alexa 488-labeled secondary antibody for the CSABP antibody, an anti-rabbit IgG-Alexa 594-labeled secondary antibody for the anti-NS3, NS4A, or NS4B antibody, and an anti-mouse IgG-Alexa 594-labeled secondary antibody for the anti-NS5A or NS5B antibody (all from Molecular Probes) respectively. After staining, the cells were examined using a confocal microscope. The results are given in FIGS. 15 and 16.

A colocalization site of CSABP (green) and KDEL molecules (red) within Huh7 cells and MH14 cells is represented by yellow in a Merge image in which the two staining images are superimposed, and as is clear from FIG. 15 such a yellow site is localized in cytoplasm, thus showing that CSABP colocalizes in the endoplasmic reticulum where KDEL is present. On the other hand, with regard to colocalization with HCV-derived protein, HCV-derived protein molecules of NS3 and below were stained only in MH14 cells that was introduced with the HCV genome, and it became clear that CSABP colocalizes with all thereof (FIG. 16). Since it is known that HCV is replicated within the endoplasmic reticulum, this result, together with the above-mentioned localization in the endoplasmic reticulum, shows that CSABP is localized in a replication site of the HCV genome.

### 2) RNA binding properties of CSABP within cells

In accordance with functional analysis on a database, CSABP is present in a DExDc domain (191st to 378th amino acids), which is reported to have RNA helicase activity, and it is therefore predicted that there are deep connections including binding properties to RNA. In order to prove that CSABP actually binds to RNA within cells, an RNA binding assay was carried out by a method by Watashi et al. (Non-Patent Document 10).
First, MH14 cells were treated with digitonin and immersed in a cell lysing solution (50 mM Tris-HCl (pH 8), 150 mM NaCl, 1 mM EDTA, 1 mM DTT, 0.5% NP-40, and Protease inhibitor tablet (Roche)) to thus lyse the cells. PolyU Sepharose (Sepharose to which uracil chain is linked) was added to a cell extract (Whole cells) obtained by subjecting the cell lysate thus obtained to centrifugation and removing an insoluble fraction, and molecules binding to RNA were recovered (pU). As a control, Protein G Sepharose specifically binding to immunoglobulin was used (G). CSABP molecules in these samples were detected by a western blot method using the above-mentioned anti-CSABP antibody. When detecting CSABP in the cell extract by the anti-CSABP antibody, in order to distinguish nonspecific bands due to an antibody, as a reference substance for CSABP detection the recombinant CSABP prepared in Example 3 was also simultaneously subjected to electrophoresis and detected.

From the results, a band was detected at around 150 kD in the Whole cells lane, and since this had the same level of molecular weight as recombinant CSABP protein (r-CSABP), it indicates that this band corresponds to CSABP protein. CSABP molecules were detected in a polyU sediment, that is, in an MH14 cell-derived substances group that had settled out by binding to RNA (FIG. 17). Since no band was observed in a Protein G Sepharose lane, nonspecific adsorption of the anti-CSABP antibody used onto Sepharose was excluded. Therefore, the band in the pU lane was formed by specific binding of the antibody, and this result shows that CSABP binds to RNA in vivo.

### 3) in vivo target molecule of CSABP

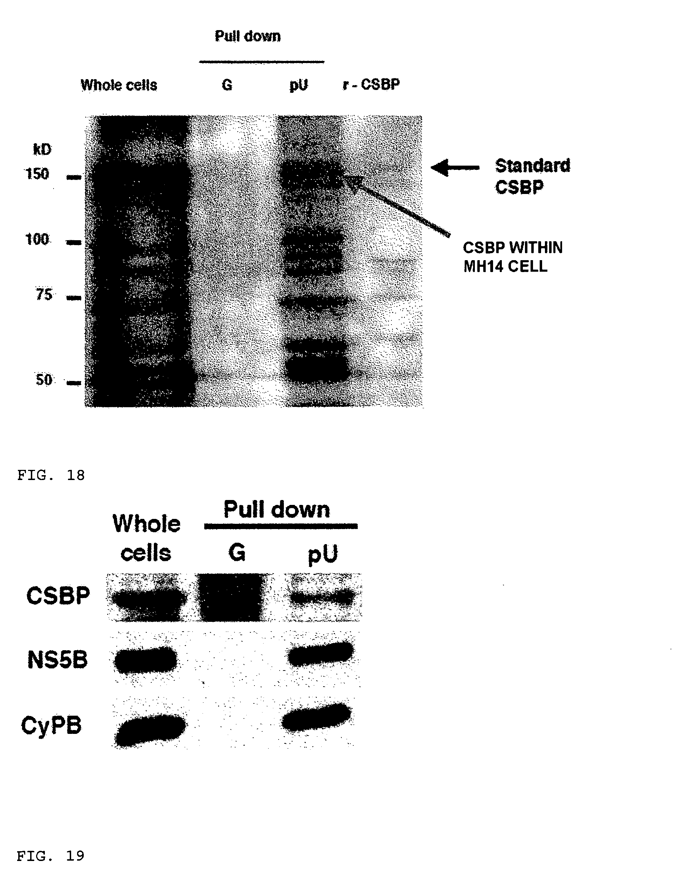

It became clear from the in vitro molecular association experiment described in (1) above that the CSABP molecule undergoes in vitro molecular association as a trimer with HCV-derived NS5B and cytoplasm-derived CyPB, and in order to confirm that this actually occurs within cells, the MH14 cell lysate was subjected to a precipitation reaction using polyU Sepharose, and the presence or absence of each protein was analyzed by a western blot method.
Specifically, each of Whole cells, pU, and G samples was prepared in the same manner as in 2) above, and CSABP, NS5B, and CyPB molecules in the samples were detected by a western blot method using an anti-CSABP polyclonal antibody, an anti-NS5B antibody, and an anti-CyPB antibody.
From the results, the respective molecules were detected in the Whole cells by the anti-CSABP antibody, the anti-NS5B antibody, and the anti-CyPB antibody. All of molecules investigated were also detected in the polyU Sepharose sediment. On the other hand, no band was detected for the Protein G Sepharose sediment (FIG. 18). These results show that CSABP also forms a trimer with NS5B and CyPB in vivo, and this trimer binds to RNA.

### 4) Effect of loss of CSABP expression on molecular association of HCV-derived protein

Changes in binding between CSABP, and HCV-derived protein and CyPB molecules via RNA by treatment using CSABP-siRNA were examined.
The siRNA treatment of MH14 cells was carried out in the same manner as in Example 5 (1). On the 3^{rd} day after siRNA introduction, the cells were collected, and an RNA binding assay was carried out in the same manner as in 2) above. The results are shown in FIG. 19. In the si-control (β-actin specific siRNA treatment) cells, the same results as in 3) above were reproduced. On the other hand, in cells treated with CSABP-siRNA (si-CSABP), hardly any CyPB band was observed. This shows that CyPB could not bind to RNA because of lack of CSABP. It is surmised that since binding of CyPB and NS5B is essential to HCV replication, dissociation of CyPB from RNA due to lack of CSABP is one factor in the HCV replication inhibition due to lack of CSABP described in Example 6.

### 5) Effect of addition of CsA on molecular association between CSABP, NS5B, and CyPB

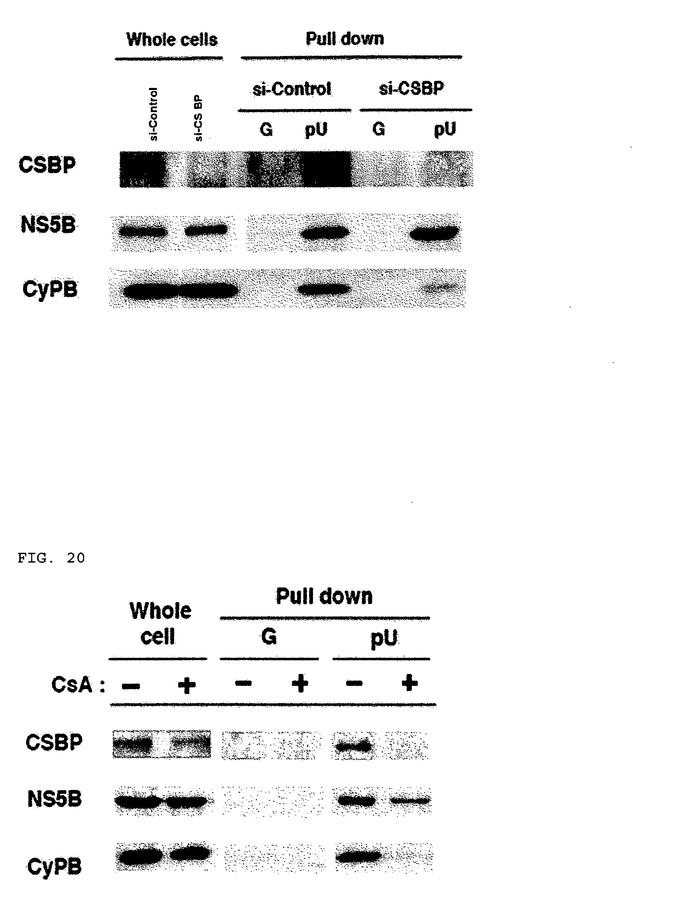

Changes in molecular association between CSABP, NS5B, and CyPB by CsA treatment were investigated.
First, 0 and 2 µg/mL of CsA was added to MH14 cells, and culturing was carried out for 24 hours. After culturing, the cells were collected, and an RNA binding assay was carried out in the same manner as in 2) above. The results are shown in FIG. 20. Following CsA treatment, binding of CSABP to RNA markedly decreased, and dissociation of CyPB from RNA was observed. These results show that both in suppression of HCV replication by CsA and in suppression of HCV replication by suppression of expression of CSABP, dissociation of CSABP and CyPB from RNA is a phenomenon common to both.

### Example 8 Determination of transcription region of CSABP gene and identification of transcription factor involved in transcription of said gene

Determination of a promoter region on the upstream of the CSABP gene and identification of a transcription factor involved in transcription of the CSABP gene were carried out using a promoter assay.

### (1) Determination of transcription region of CSABP gene

BAC clone number RP11-64F7, which is a genome clone containing a CSABP gene coded on chromosome 5 and has been identified on the NCBI database, was purchased from the Children's Hospital Oakland Research Institute. In order to determine a promoter region, a few types of cDNAs in the CSABP upstream region were subcloned by PCR, and these cDNAs were inserted into luciferase vectors (Promega). First, in order to amplify the base -4292 to +2414 region of the CSABP gene (here, A of the initiation codon ATG of same gene being defined as base +1 and the base one base upstream (5' side) of ATG being defined as base -1), CSABP -4292 forward primer: 5'-GTCCCAGGAGACAACTCCAT-3' (SEQ ID No: 24) and CSABP +2414 reverse primer: 5'-CATCAGATCCTTGACTTGGTAGG-3' (SEQ ID No: 25) were synthesized, and PCR was carried out using the above-mentioned BAC as a template. This fragment was inserted into a pCRII-TOPO vector (Invitrogen) by a TOPO TA Cloning^{®} kit (Invitrogen). CSABP bases -1519 to -44 and CSABP bases -4292 to -1516 were cut out by cleaving this vector with NheI and with SacI and NheI respectively and inserted into pGL4.10 luciferase vectors (Promega).

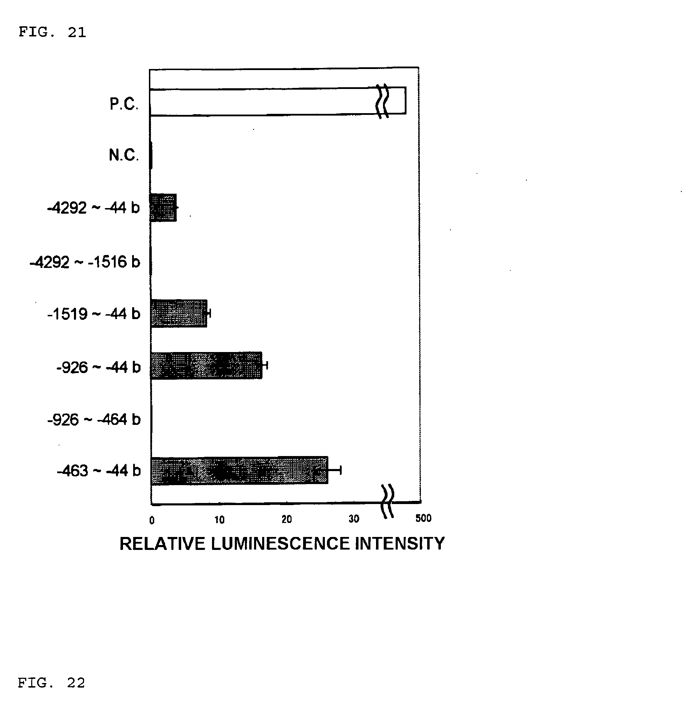

Furthermore, in order to amplify each of CSABP base -926 to -44, base -926 to -464, and base -463 to -44 regions, primers with added SacI and HindIII recognition sequences (CSABP -926 forward primer: 5'-TTTGAGCTCAATATTCCGCCCCCTTCTTA-3' (SEQ ID No: 26), CSABP -44 reverse primer: 5'-TTTAAGCTTGCTAGCTGACAGCCAATGAC-3' (SEQ ID No: 27), CSABP -464 reverse primer: 5'-TTTAAGCTTAGGTTCCTACCTTCATGGAGTT-3' (SEQ ID No: 28), and CSABP -463 forward primer: 5'-TTTGAGCTCTGGCTCTCTGGTTCTCCAAT-3' (SEQ ID No: 29) (the underlining denoting restriction enzyme recognition sequences)) were synthesized, and PCR was carried out using the above-mentioned fragment (CSABP bases -1519 to -44) as a template. Each of the fragments thus amplified was inserted into a pGL4.10 luciferase vector (Promega). These vectors were introduced together with the pGL4.74 luciferase vector (Promega) into the Huh-7 liver cancer cell line by LF2000 (Invitrogen), and 24 hours thereafter the cells were collected. Luminescence of the luciferase in the cells thus collected was measured by a Lumincescencer PSN (Atto) in accordance with the Dual-Luciferase^{®} Reporter Assay System protocol (Cat.E1910, Promega). In addition, a pGL4.13 luciferase vector (Promega) having SV40 early enhancer/promoter inserted was used as a positive control, and a pGL4.10 luciferase vector having none inserted was used as a negative control. From the results, luminescence activity was observed in the base -463 to -44 region (SEQ ID No: 30) (FIG. 21), and it became clear that this region is a promoter region (FIG. 22).

### (2) Identification of transcription factor

As a result of looking up the above-mentioned promoter region on the TFSEARCH database, it has been found that transcription factor CREB-, GATA- and AP-1-binding motifs are present (TGACGTCA (bases -220 to -213), CGTGATATCA (bases -188 to -179), and GGTGACTGAGG (bases -150 to -140) respectively). Among these transcription factors, in order to clarify one that is important for expression of CSABP, mutations were introduced into the three transcription factor-binding motifs present within the region using primers shown in the table below, thus preparing a mutant CREB-binding motif cDNA (m-CREB), a mutant GATA-binding motif cDNA (*m*-GATA), and a mutant AP-1-binding motif cDNA (*m*-AP-1).

**[Table 3]**

| Primer name | Sequence |
|---|---|
| CSBP. m-CREB F | CGCCGGCCCTGGTGTGGTCATTACGCCCGC (SEQ ID NO: 31) |
| CSBP. m-CREB R | GCGGGCGTAATGACCACACCAGGGCCGGCG (SEQ ID NO: 32) |
| CSBP. m-GATA F | CTCGCCTGGCCGTTGTATCAATGGCGCAGG (SEQ ID NO: 33) |
| CSBP. m-GATA R | CCTGCGCCATTGATACAACGGCCAGGCGAG (SEQ ID NO: 34) |
| CSBP. m-AP-1 F | ACTTCTGCTGTGGCGGTAGTTGAGGCCTTTCTGGTGA (SEQ ID NO: 35) |
| CSBP. m-AP-1 R | TCACCAGAAAGGCCTCAACTACCGCCACAGCAGAAGT (SEQ ID NO: 36) |

Mutation sites in the above-mentioned mutant cDNAs are shown in the table below. In the table, wild types prior to mutagenesis are denoted by w-CREB, w-GATA, and w-AP-1.

**[Table 4]**

| cDNA Name | Mutation site |
|---|---|
| w-CREB | TGACGTCA |
| m-CREB | --TG---- |
| w-GATA | CGTGATATCA |
| m-GATA | ---TG----- |
| w-AP-1 | GGTGACTGAGG |
| m-AP-1 | ---ATG----- |

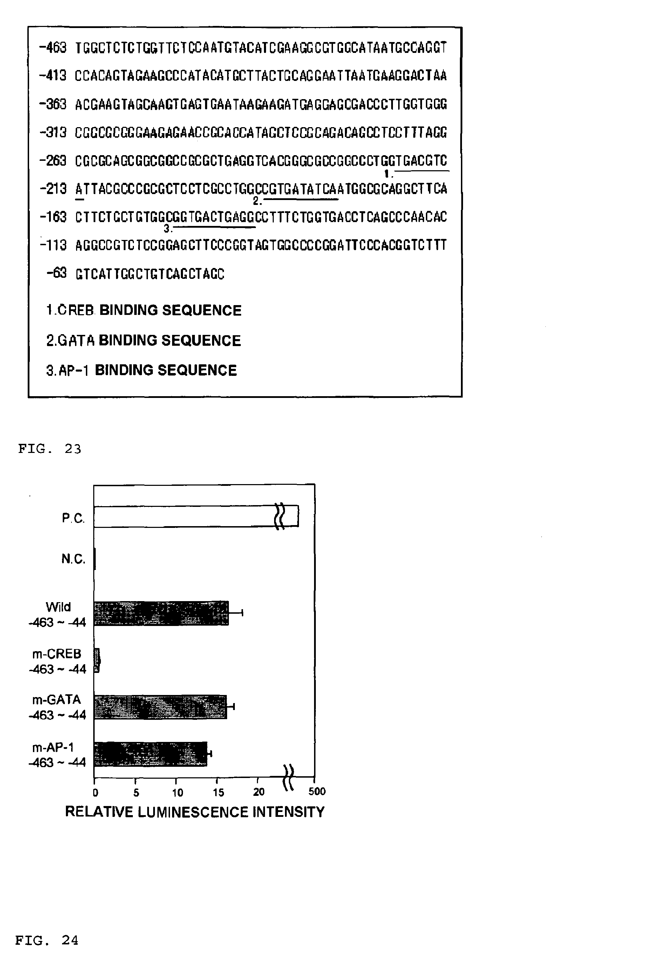

Mutant cDNAs thus obtained were introduced into Huh-7 in the same manner as in (1) above, and luminescence was measured. From the results, a marked decrease in luciferase activity was only observed when *m*-CREB cDNA was introduced (FIG. 23). This suggests that a transcription factor belonging to the CREB/ATF family binding to the CRE motif (TGACGTCA) is involved in transcription of CSABP.

### Example 9 Control of expression of CSABP by anti-methylating agent

### (1) DNA methylation of promoter region of CSABP gene

The promoter region (bases -463 to -44 (SEQ ID No: 30)) of the CSABP gene identified in Example 8 was analyzed by a CpG island analysis tool (CpG Island Searcher, http://cpgislands.usc.edu/), and the results show that this region is a CpG island (FIG. 24). The CpG sequence is a sequence that is subject to DNA methylation, and when the CpG island in the promoter region is subjected to DNA methylation, expression of the gene is thought to be suppressed (see Bernstein et al., 2007, supra). In order to analyze the relationship between CSABP gene expression and DNA methylation, expression of the CSABP gene after 5-aza-2'-deoxycytidine, which is a DNA methylation inhibitor, was administered was analyzed by an RT-PCR method.

### (2) Effect of anti-methylating agent on CSABP expression

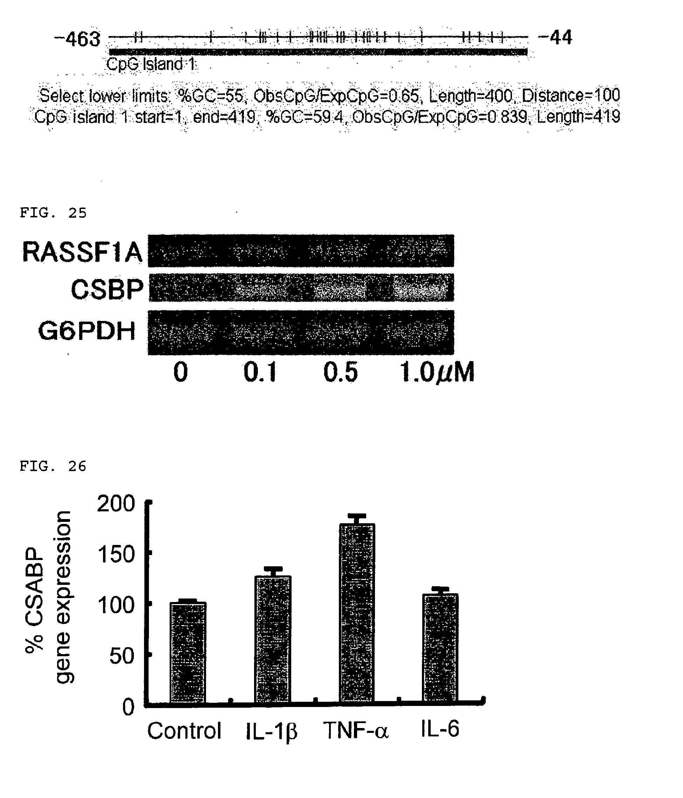

10⁵ cells/well of MH14 cells (liver cancer cell line) were precultured in a 6 well plate with MH14 cell culture medium for 24 hours, and 5-aza-2'-deoxycytidine (Sigma) was then added thereto. Subsequently, medium exchange was carried out every 24 hours with a medium to which the methylation inhibitor had been added, and the cells were collected after 4 days. The amount of 5-aza-2'-deoxycytidine added was 0, 0.1, 0.5, and 1.0 µM. Total RNA was recovered from the cells thus collected in accordance with a standard method using an RNeasy kit (Qiagen, Cat. 74104). This total RNA was subjected to a reverse transcription reaction in accordance with a standard method using a Transcriptor First Strand cDNA Synthesis Kit (Roche, Cat. 04379012001), and the cDNA thus obtained was used as a template. The primer employed the above-mentioned CSABP primer (SEQ ID Nos: 5 and 6), and a PCR reaction was carried out in a Gotaq^{®} PCR system (Promega) at 95°C, 5 minutes × 1 cycle, 95°C, 0.5 minutes, 55°C, 0.5 minutes, 72°C, 2.5 minutes × 35 cycles, 72°C, 7 minutes × 1 cycle. As a positive control, a reaction was carried out in the same manner using the RASSF1A primers RASSF1A transcript variant D 391 to 415 forward primer: 5'-CAGATTGCAAGTTCACCTGCCACTA-3' (SEQ ID No: 38) and RASSF1A transcript variant D 635 to 660 reverse primer: 5'-GATGAAGCCTGTGTAAGAACCGTCCT-3' (SEQ ID No: 39), which is a gene for which a relationship between expression and DNA methylation in a promoter region has been shown by Cui X et al., Hum Pathol. 2006; 37 (3): 298-311. As an internal control, a reaction was carried out in the same manner using the above-mentioned G6PDH primer (SEQ ID Nos: 20 and 21). The results are shown in FIG. 25.

The amount of expression of G6PDH was uniform within a test section, and no cytotoxicity was observed by addition of the agent at the concentrations used here. Since expression of RASSF1A, which is a positive control, was increased by the addition of 5-aza-2'-deoxycytidine, it was confirmed that this agent was acting effectively. The amount of expression of the CSABP gene showed an increase due to the addition of 5-aza-2'-deoxycytidine that was concentration-dependent. This shows that when the degree of methylation of DNA decreases, expression of the CSABP gene is increased, that is, expression of the CSABP gene is highly related to methylation of DNA.

### Example 10 Induction of expression of CSABP by inflammatory cytokine

Normal human liver cells (Primary Human Liver Hepatocyte Cells, DS PHARMA BIOMEDICAL CO., LTD., Cat. No. ABI-3716) were cultured in a 6 well plate at a density of 2 × 10⁵ cells/well, and human IL-1β, TNFα, or IL-6 (all from BioSource) was added thereto to give a final concentration in the well of 1 pg/mL, 100 pg/mL, or 1 ng/mL respectively. 8 hours after the addition, the cells were collected, and expression of CSABP within the cells was quantitatively determined by real time PCR in the same manner as in Example 5 (2). The results are shown in FIG. 26.
The amount of expression of CSABP was increased by about 1.8 times compared with the control by the addition of 100 pg/mL of TNF-α to normal liver cells. Furthermore, some increase was observed for other inflammatory cytokines (IL-1β and IL-6). The results of Example 2 above show that expression of CSABP in normal cells is undetectable or very low, but it is very surprising that expression of CABP in such normal cells is induced by the action of inflammatory cytokines.
The above-mentioned results suggest, while taking into consideration a report that secretion of TNF-α within liver tissue is increased during HCV infection (see e.g. Tilg H et al. Gastroenterol 103 (1): 264-274, 1992 and Gonzalez-Amaro R et al. J Exp Med 179 (3): 841-848, 1994), the possibility that HCV advantageously promotes replication by utilizing inducibly expressed CSABP, since inflammation is caused within liver tissue at an early stage of HCV infection, and expression of CSABP is induced by TNF-α from immunocompetent cells. Furthermore, the above-mentioned results show that the occurrence of inflammation induces expression of CSABP in cells at the inflamed site, thus creating an environment in which a virus, in particular an RNA virus such as HCV, in the replication of which CSABP is involved, easily replicates; it is suggested that by suppressing the expression of CSABP under such circumstances it is possible to prevent viral growth particularly effectively, and by administering an agent, etc. that suppresses expression of CSABP to a subject at an initial stage of inflammation and, furthermore, to a subject for which there is a risk of inflammation occurring, it is possible to inhibit creation of the above-mentioned environment in which a virus easily replicates, thus enabling a viral disease to be prevented effectively.

## Claims

1. A composition for treating a CSABP-related disease, the composition comprising a component that controls the abundance and/or function of a CSABP.

2. The composition according to Claim 1, wherein the component that controls the abundance and/or function of a CSABP is selected from the group consisting of a CSABP, a functional variant of a CSABP, a dominant negative variant of a CSABP, NS5B, cyclophilin B, and CSABP-binding variants thereof, an anti-CSABP antibody, nucleic acids coding therefor, vectors expressing these, and a substance that controls CSABP gene expression.

3. A composition for diagnosing a CSABP-related disease, the composition comprising a component that detects a CSABP-coding nucleic acid or an expression product thereof.

4. The composition according to Claim 3, wherein the component that detects a CSABP-coding nucleic acid or an expression product thereof is selected from the group consisting of an anti-CSABP antibody, cyclosporin A and a derivative thereof, NS5B, cyclophilin B, and CSABP-binding variants thereof, and a nucleic acid that specifically hybridizes with a CSABP-coding nucleic acid, a unique fragment thereof, a transcription product of said nucleic acid, or a splice product.

5. The composition according to any one of Claims 1 to 4, wherein the CSABP-related disease is selected from the group consisting of an RNA virus infection, hepatitis C, an inflammatory disease, a state in which replication of an RNA virus is promoted, and a tumor.

6. A method for screening a component that detects and/or controls a CSABP, the method comprising a step of making a test substance coexist with a CSABP or a fragment thereof.

7. A composition for controlling the growth of an RNA virus, the composition comprising a component that controls the abundance and/or function of a CSABP.

8. A composition for detecting and/or inhibiting cyclosporin A and a derivative thereof, NS5B, or cyclophilin B, the composition comprising a CSABP or a functional variant thereof.

9. A method for screening a substance that controls CSABP gene expression, the method comprising 1) a step of making a cell having a nucleic acid molecule containing a CSABP promoter and a reporter sequence operably linked thereto coexist with a test substance, and 2) a step of detecting an expression product of the reporter sequence.

10. A method for screening a substance that controls CSABP gene expression, the method comprising a step of recovering a substance that binds to a CSABP promoter.

11. A CSABP promoter, selected from the group consisting of
i) a nucleic acid molecule containing a base sequence represented by SEQ ID No: 37 or a portion thereof,
ii) a nucleic acid molecule that has one or more mutations in the base sequence of nucleic acid molecule i) but that still has equivalent functions to said nucleic acid molecule,
iii) a nucleic acid molecule that hybridizes with a complementary strand of nucleic acid molecule i) or ii) or a fragment thereof under stringent conditions and that has equivalent functions to said nucleic acid molecule, and
iv) a nucleic acid molecule that has at least 70% homology to nucleic acid molecule i) or ii) and that has equivalent functions to said nucleic acid molecule.

12. A vector having a CSABP promoter.

13. A cell having a nucleic acid molecule containing a CSABP promoter and a reporter sequence operably linked thereto.

14. A method for determining the onset or disease stage of a CSABP-related disease, the method comprising a step of detecting methylation in a CSABP gene.


SEQUENCE LISTING

<110> Sapporo Medical University
Tokyo University of Science
Kyoto University
Sapporo Immuno Diagnostic Laboratory

<120> Cyclosporin A binding protein

<130> PCT2326SI

<140> EP08828474.0
<141> 2008-08-22

<150> JP2007-217755
<151> 2007-08-24

<160> 39

<170> PatentIn version 3.5

<210> 1
<211> 1430
<212> PRT
<213> Homo sapiens

<400> 1

Met Ser Arg Pro Ser Ser Val Ser Pro Arg Gln Pro Ala Pro Gly Gly
1               5                   10                  15

Gly Gly Gly Gly Gly Pro Ser Pro Cys Gly Pro Gly Gly Gly Gly Arg
            20                  25                  30

Ala Lys Gly Leu Lys Asp Ile Arg Ile Asp Glu Glu Val Lys Ile Ala
        35                  40                  45

Val Asn Ile Ala Leu Glu Arg Phe Arg Tyr Gly Asp Gln Arg Glu Met
    50                  55                  60

Glu Phe Pro Ser Ser Leu Thr Ser Thr Glu Arg Ala Phe Ile His Arg
65                  70                  75                  80

Leu Ser Gln Ser Leu Gly Leu Val Ser Lys Ser Lys Gly Lys Gly Ala
                85                  90                  95

Asn Arg Tyr Leu Thr Val Lys Lys Lys Asp Gly Ser Glu Thr Ala His
            100                 105                 110

Ala Met Met Thr Cys Asn Leu Thr His Asn Thr Lys His Ala Val Arg
        115                 120                 125

Ser Leu Ile Gln Arg Phe Pro Val Thr Asn Lys Glu Arg Thr Glu Leu
    130                 135                 140

Leu Pro Lys Thr Glu Arg Gly Asn Val Phe Ala Val Glu Ala Glu Asn
145                 150                 155                 160

Arg Glu Met Ser Lys Thr Ser Gly Arg Leu Asn Asn Gly Ile Pro Gln
                165                 170                 175

Ile Pro Val Lys Arg Gly Glu Ser Glu Phe Asp Ser Phe Arg Gln Ser
            180                 185                 190

Leu Pro Val Phe Glu Lys Gln Glu Glu Ile Val Lys Ile Ile Lys Glu
        195                 200                 205

Asn Lys Val Val Leu Ile Val Gly Glu Thr Gly Ser Gly Lys Thr Thr
    210                 215                 220

Gln Ile Pro Gln Phe Leu Leu Asp Asp Cys Phe Lys Asn Gly Ile Pro
225                 230                 235                 240

Cys Arg Ile Phe Cys Thr Gln Pro Arg Arg Leu Ala Ala Ile Ala Val
                245                 250                 255

Ala Glu Arg Val Ala Ala Glu Arg Arg Glu Arg Ile Gly Gln Thr Ile
            260                 265                 270

Gly Tyr Gln Ile Arg Leu Glu Ser Arg Val Ser Pro Lys Thr Leu Leu
        275                 280                 285

Thr Phe Cys Thr Asn Gly Val Leu Leu Arg Thr Leu Met Ala Gly Asp
    290                 295                 300

Ser Thr Leu Ser Thr Val Thr His Val Ile Val Asp Glu Val His Glu
305                 310                 315                 320

Arg Asp Arg Phe Ser Asp Phe Leu Leu Thr Lys Leu Arg Asp Leu Leu
                325                 330                 335

Gln Lys His Pro Thr Leu Lys Leu Ile Leu Ser Ser Ala Ala Leu Asp
            340                 345                 350

Val Asn Leu Phe Ile Arg Tyr Phe Gly Ser Cys Pro Val Ile Tyr Ile
        355                 360                 365

Gln Gly Arg Pro Phe Glu Val Lys Glu Met Phe Leu Glu Asp Ile Leu
    370                 375                 380

Arg Thr Thr Gly Tyr Thr Asn Lys Glu Met Leu Lys Tyr Lys Lys Glu
385                 390                 395                 400

Lys Gln Gln Glu Glu Lys Gln Gln Thr Thr Leu Thr Glu Trp Tyr Ser
                405                 410                 415

Ala Gln Glu Asn Ser Phe Lys Pro Glu Ser Gln Arg Gln Arg Thr Val
            420                 425                 430

Leu Asn Val Thr Asp Glu Tyr Asp Leu Leu Asp Asp Gly Gly Asp Ala
        435                 440                 445

Val Phe Ser Gln Leu Thr Glu Lys Asp Val Asn Cys Leu Glu Pro Trp
    450                 455                 460

Leu Ile Lys Glu Met Asp Ala Cys Leu Ser Asp Ile Trp Leu His Lys
465                 470                 475                 480

Asp Ile Asp Ala Phe Ala Gln Val Phe His Leu Ile Leu Thr Glu Asn
                485                 490                 495

Val Ser Val Asp Tyr Arg His Ser Glu Thr Ser Ala Thr Ala Leu Met
            500                 505                 510

Val Ala Ala Gly Arg Gly Phe Ala Ser Gln Val Glu Gln Leu Ile Ser
        515                 520                 525

Met Gly Ala Asn Val His Ser Lys Ala Ser Asn Gly Trp Met Ala Leu
    530                 535                 540

Asp Trp Ala Lys His Phe Gly Gln Thr Glu Ile Val Asp Leu Leu Glu
545                 550                 555                 560

Ser Tyr Ser Ala Ser Leu Glu Phe Gly Asn Leu Asp Glu Ser Ser Leu
                565                 570                 575

Val Gln Thr Asn Gly Ser Asp Leu Ser Ala Glu Asp Arg Glu Leu Leu
            580                 585                 590

Lys Ala Tyr His His Ser Phe Asp Asp Glu Lys Val Asp Leu Asp Leu
        595                 600                 605

Ile Met His Leu Leu Tyr Asn Ile Cys His Ser Cys Asp Ala Gly Ala
    610                 615                 620

Val Leu Ile Phe Leu Pro Gly Tyr Asp Glu Ile Val Gly Leu Arg Asp
625                 630                 635                 640

Arg Ile Leu Phe Asp Asp Lys Arg Phe Ala Asp Asn Thr His Arg Tyr

                645                 650                 655

Gln Val Phe Met Leu His Ser Asn Met Gln Thr Ser Asp Gln Lys Lys
            660                 665                 670

Val Leu Lys Asn Pro Pro Ala Gly Val Arg Lys Ile Ile Leu Ser Thr
        675                 680                 685

Asn Ile Ala Glu Thr Ser Ile Thr Val Asn Asp Val Val Phe Val Ile
    690                 695                 700

Asp Ser Gly Lys Val Lys Glu Lys Ser Phe Asp Ala Leu Asn Phe Val
705                 710                 715                 720

Thr Met Leu Lys Met Val Trp Ile Ser Lys Ala Ser Ala Ile Gln Arg
                725                 730                 735

Lys Gly Arg Ala Gly Arg Cys Arg Pro Gly Ile Cys Phe Arg Leu Phe
            740                 745                 750

Ser Arg Leu Arg Phe Gln Asn Met Leu Glu Phe Gln Thr Pro Glu Leu
        755                 760                 765

Leu Arg Met Pro Leu Gln Glu Leu Cys Leu His Thr Lys Leu Leu Ala
    770                 775                 780

Pro Val Asn Cys Pro Ile Ala Asp Phe Leu Met Lys Ala Pro Glu Pro
785                 790                 795                 800

Pro Pro Ala Leu Ile Val Arg Asn Ala Val Gln Met Leu Lys Thr Ile
                805                 810                 815

Asp Ala Met Asp Thr Trp Glu Asp Leu Thr Glu Leu Gly Tyr His Leu
            820                 825                 830

Ala Asp Leu Pro Val Glu Pro His Leu Gly Lys Met Val Leu Cys Ala
        835                 840                 845

Val Val Leu Lys Cys Leu Asp Pro Ile Leu Thr Ile Ala Cys Thr Leu
    850                 855                 860

Ala Tyr Arg Asp Pro Phe Val Leu Pro Thr Gln Ala Ser Gln Lys Arg
865                 870                 875                 880

Ala Ala Met Leu Cys Arg Lys Arg Phe Thr Ala Gly Ala Phe Ser Asp
                885                 890                 895

His Met Ala Leu Leu Arg Ala Phe Gln Ala Trp Gln Lys Ala Arg Ser
            900                 905                 910

Asp Gly Trp Glu Arg Ala Phe Cys Glu Lys Asn Phe Leu Ser Gln Ala
        915                 920                 925

Thr Met Glu Ile Ile Ile Gly Met Arg Thr Gln Leu Leu Gly Gln Leu
    930                 935                 940

Arg Ala Ser Gly Phe Val Arg Ala Arg Gly Gly Gly Asp Ile Arg Asp
945                 950                 955                 960

Val Asn Thr Asn Ser Glu Asn Trp Ala Val Val Lys Ala Ala Leu Val
                965                 970                 975

Ala Gly Met Tyr Pro Asn Leu Val His Val Asp Arg Glu Asn Leu Val
            980                 985                 990

Leu Thr Gly Pro Lys Glu Lys Lys  Val Arg Phe His Pro  Ala Ser Val
        995                 1000                 1005

Leu Ser  Gln Pro Gln Tyr Lys  Lys Ile Pro Pro Ala  Asn Gly Gln
    1010                 1015                 1020

Ala Ala  Ala Ile Lys Ala Leu  Pro Thr Asp Trp Leu  Ile Tyr Asp
    1025                 1030                 1035

Glu Met  Thr Arg Ala His Arg  Ile Ala Asn Ile Arg  Cys Cys Ser
    1040                 1045                 1050

Ala Val  Thr Pro Val Thr Ile  Leu Val Phe Cys Gly  Pro Ala Arg
    1055                 1060                 1065

Leu Ala  Ser Asn Ala Leu Gln  Glu Pro Ser Ser Phe  Arg Val Asp
    1070                 1075                 1080

Gly Ile  Pro Asn Asp Ser Ser  Asp Ser Glu Met Glu  Asp Lys Thr
    1085                 1090                 1095

Thr Ala  Asn Leu Ala Ala Leu  Lys Leu Asp Glu Trp  Leu His Phe
    1100                 1105                 1110

Thr Leu  Glu Pro Glu Ala Ala  Ser Leu Leu Leu Gln  Leu Arg Gln
    1115                 1120                 1125

Lys Trp  His Ser Leu Phe Leu  Arg Arg Met Arg Ala  Pro Ser Lys
    1130                 1135                 1140

Pro Trp Ser Gln Val Asp Glu Ala Thr Ile Arg Ala Ile Ile Ala
    1145                1150                1155

Val Leu Ser Thr Glu Glu Gln Ser Ala Gly Leu Gln Gln Pro Ser
    1160                1165                1170

Gly Ile Gly Gln Arg Pro Arg Pro Met Ser Ser Glu Glu Leu Pro
    1175                1180                1185

Leu Ala Ser Ser Trp Arg Ser Asn Asn Ser Arg Lys Ser Ser Ala
    1190                1195                1200

Asp Thr Glu Phe Ser Asp Glu Cys Thr Thr Ala Glu Arg Val Leu
    1205                1210                1215

Met Lys Ser Pro Ser Pro Ala Leu His Pro Pro Gln Lys Tyr Lys
    1220                1225                1230

Asp Arg Gly Ile Leu His Pro Lys Arg Gly Thr Glu Asp Arg Ser
    1235                1240                1245

Asp Gln Ser Ser Leu Lys Ser Thr Asp Ser Ser Ser Tyr Pro Ser
    1250                1255                1260

Pro Cys Ala Ser Pro Ser Pro Pro Ser Ser Gly Lys Gly Ser Lys
    1265                1270                1275

Ser Pro Ser Pro Arg Pro Asn Met Pro Val Arg Tyr Phe Ile Met
    1280                1285                1290

Lys Ser Ser Asn Leu Arg Asn Leu Glu Ile Ser Gln Gln Lys Gly
    1295                1300                1305

Ile Trp Ser Thr Thr Pro Ser Asn Glu Arg Lys Leu Asn Arg Ala
    1310                1315                1320

Phe Trp Glu Ser Ser Ile Val Tyr Leu Val Phe Ser Val Gln Gly
    1325                1330                1335

Ser Gly His Phe Gln Gly Phe Ser Arg Met Ser Ser Glu Ile Gly
    1340                1345                1350

Arg Glu Lys Ser Gln Asp Trp Gly Ser Ala Gly Leu Gly Gly Val
    1355                1360                1365

Phe Lys Val Glu Trp Ile Arg Lys Glu Ser Leu Pro Phe Gln Phe
    1370                1375                1380

Ala His His Leu Leu Asn Pro Trp Asn Asp Asn Lys Lys Val Gln
    1385                1390                1395

Ile Ser Arg Asp Gly Gln Glu Leu Glu Pro Leu Val Gly Glu Gln
    1400                1405                1410

Leu Leu Gln Leu Trp Glu Arg Leu Pro Leu Gly Glu Lys Asn Thr
    1415                1420                1425

Thr Asp
    1430

<210> 2
<211> 6301
<212> DNA
<213> Homo sapiens

<400> 2
tatcaatggc gcaggcttca cttctgctgt ggcggtgact gaggcctttc tggtgacctc 60

agcccaacac aggccgtctc cggagcttcc cggtagtggc cccggattcc cacggtcttt 120

gtcattggct gtcagctagc aggcctggcc gctcccgtgc ggagagacca tctcttcagg 180

gcaatgtcca ggccgagcag cgtctccccg cggcagccgg ctcctggcgg tggcggaggc 240

ggcggcccct cgccttgtgg ccctgggggc ggcggccggg ccaaggggct gaaggacatt 300

cgcattgatg aggaggtgaa gatcgcagtc aatatcgcgc tggagcgctt ccgatacggg 360

gaccagagag aaatggaatt tccttcttct ttgaccagta ctgaaagagc ctttattcat 420

cgactcagtc agtctcttgg tttggtctct aaaagtaaag gaaagggagc aaatagatac 480

ctaactgtga agaagaaaga tggatcagaa acagctcatg caatgatgac ctgtaatttg 540

actcataata caaaacatgc tgttaggagc ctaattcaaa gatttcctgt caccaataaa 600

gagcgtacag aacttctgcc taaaacagaa agaggaaatg tgtttgcagt tgaagctgaa 660

aaccgggaaa tgagcaagac aagtgggcga ctcaacaatg gcatacctca gattccagtg 720

aaaagaggag aatccgaatt tgattctttt aggcagtctt taccagtgtt tgagaaacag 780

gaagaaattg ttaaaataat taaggaaaat aaagtagttt tgattgtagg agaaactggg 840

tctggaaaga ccacacagat tcctcagttc cttttagatg attgctttaa aaatggtatc 900

ccctgccgta tattttgtac tcaaccaaga cgattggcag ctatcgctgt ggctgaaaga 960

gttgccgcag agagacggga aaggattggt caaacaattg gttatcagat ccgattagaa 1020

agcagggttt ctccaaagac acttctgaca ttttgtacta atggggtatt gcttcgtaca 1080

ttgatggcag gagatagtac gttgtcgact gtgacacatg ttatcgtgga tgaagtgcat 1140

gaaagggatc gatttagtga ttttttactt acaaagttaa gagatttgtt gcaaaagcac 1200

ccaactttga aactaattct ttctagtgct gccttggatg taaatctctt tataagatat 1260

tttggaagtt gtccagtgat atatatacag ggaagaccat ttgaagtaaa agaaatgttt 1320

ctggaagata ttttaagaac aactggatat acaaacaaag aaatgttaaa atataaaaag 1380

gaaaaacagc aagaagagaa acaacaaacc acacttacag aatggtactc agctcaagaa 1440

aatagtttca agcctgaatc tcagaggcag agaactgttc taaatgtgac tgatgagtat 1500

gacttactgg atgatggtgg tgatgctgtc ttcagtcagc tgactgaaaa agatgtgaat 1560

tgccttgaac catggttaat aaaggaaatg gatgcttgcc tttctgatat atggctacat 1620

aaagatattg atgcctttgc tcaggtcttt catctcattt taactgaaaa tgttagtgtt 1680

gattacagac atagtgaaac cagtgcaaca gctctgatgg ttgctgcagg acgtggcttt 1740

gcaagtcaag tagaacagtt aatcagtatg ggagccaatg tccatagtaa agcatcaaat 1800

ggctggatgg cattggattg ggctaaacac tttgggcaga ctgaaattgt ggatcttcta 1860

gaatcttaca gtgcttcact ggaatttgga aatctagatg aaagttctct ggttcaaaca 1920

aatggaagtg acctcagtgc tgaagacaga gagctcctga aagcttatca tcatagtttc 1980

gatgatgaaa aagtagactt ggatttgatc atgcatcttc tatacaatat ctgccatagt 2040

tgtgatgctg gtgcagtact aatttttctg cctggatatg acgaaattgt tggactgaga 2100

gatcgcatcc tgtttgatga caagcggttt gctgacaata cacatagata ccaagtcttt 2160

atgcttcatt caaatatgca aacatccgat caaaagaaag tattaaaaaa cccacctgca 2220

ggtgttcgaa aaataattct ttccaccaat attgctgaaa ccagcatcac agtcaatgat 2280

gttgtctttg ttattgattc tggtaaggtg aaagagaaat cctttgatgc tctgaatttt 2340

gttacaatgt taaaaatggt atggatttcc aaagctagtg ccatacagcg gaaaggcagg 2400

gcagggcgat gtagacctgg aatttgtttt cgtctgttca gtagactccg attccagaat 2460

atgttggaat ttcagactcc ggaacttttg agaatgccat tacaggaact ttgcttacat 2520

accaagctgt tagccccagt taattgtccc attgctgatt ttcttatgaa agctcctgaa 2580

cctccaccag ctttaattgt aagaaatgct gtacaaatgc ttaagacaat agatgcaatg 2640

gatacatggg aagatctgac tgaacttggg tatcatttgg ctgacttgcc agtagaacca 2700

catcttggta aaatggtctt gtgtgctgtt gttttaaagt gtctggaccc catccttaca 2760

attgcttgca cactagctta tcgagatcct tttgtactac ctactcaggc ctctcaaaaa 2820

cgtgcagcta tgctttgtag gaaacgtttt actgcaggag ctttcagtga ccatatggca 2880

cttctcagag cattccaggc ctggcaaaaa gcacgaagtg atgggtggga gcgagccttt 2940

tgtgaaaaga attttctttc acaggctact atggaaataa tcataggcat gagaacacag 3000

ttgcttggtc aacttagagc atcaggtttt gttagagcac gaggtggtgg tgacattcgg 3060

gacgttaaca caaactctga gaattgggct gtcgttaaag ctgcattggt ggcaggcatg 3120

tatcctaatt tagtccacgt ggacagagag aatctagtgt tgacagggcc aaaggagaaa 3180

aaagtacgat ttcatcctgc ttcagttctc agtcagcctc aatataaaaa gattcctcca 3240

gccaatggtc aagctgcagc aattaaggca ctgcccacag attggcttat ttatgatgaa 3300

atgaccagag cccatagaat agctaatatt agatgttgtt cagcagtgac gcctgtcact 3360

atattggtat tctgtggacc agctagattg gcaagtaatg ctcttcagga accttcatcc 3420

tttagagtgg atggcattcc caatgacagt agtgatagtg aaatggagga caaaactaca 3480

gctaatttgg cagccttgaa acttgatgag tggctccatt tcacactgga gccagaggca 3540

gctagtttat tgctgcagct cagacagaag tggcatagct tatttttacg ccgaatgaga 3600

gctccatcta aaccttggtc tcaagttgat gaagctacca taagagcaat tatagctgtt 3660

ttaagcactg aagaacagtc tgcaggttta caacaaccat ctgggattgg ccaaaggcca 3720

aggcctatgt cttcagaaga gcttcctttg gcctcatctt ggaggtcaaa taatagtagg 3780

aaaagttcag cagatactga attttctgat gagtgtacta ctgcagaaag agtactgatg 3840

aaatctccat ctccagcatt acacccacct cagaagtaca aagatagagg aattttacat 3900

cctaaacgag gtactgagga ccgatcagat cagtcttctc tgaaatctac agacagcagt 3960

agttacccaa gtccttgtgc tagtccttct cctccatcct caggaaaggg ctcaaaatct 4020

ccttcgccaa gaccaaacat gcctgttcga tacttcataa tgaagagtag caatttgaga 4080

aaccttgaaa tttctcaaca gaagggtatc tggtctacaa ctcctagtaa tgaacggaag 4140

ctaaatcgag ccttttggga aagcagcata gtttacttgg tattttctgt tcaaggatct 4200

ggacatttcc agggattttc taggatgtct tctgagattg gaagggaaaa gagtcaggac 4260

tggggctctg ctggactagg aggagtattt aaggtggagt ggatacgaaa agaaagcctt 4320

ccctttcaat ttgcacacca tttactcaat ccatggaatg acaacaagaa agtgcagata 4380

agcagggatg ggcaggaact agaacctctg gttggtgaac agttgctcca gttatgggaa 4440

cgtcttccct tgggagaaaa aaacacaact gattgacact cagaactctt agctgtggaa 4500

gaacatcatg cctatttata accactgaat gcactgactt tcaaaaactg aggtggggtg 4560

tgtgttacga atgggctttt taacactttt agagtgttgc tttagaacta ccatcttcat 4620

atacaggaga aaggaagcat ttaaattttt atagtgatta tagagaatga ttatatgatg 4680

tttgtaatga ataaaatagt agtttcatta tttggcacaa tagcagttta ttttaaacaa 4740

acaatttgaa gttaaacatt tcatttttaa aaacactgaa ttacagttct tattgatgac 4800

tttttaatgc agagtaagtt gtttaagaaa ggcctgaata tatcagaaca tctgagcacc 4860

attttagaag agtcaactct taagattatc attagaaaac aaatacgtca acacctatga 4920

aaagaagcgg ggaaaacaac aacaaggaca acaacaaaaa accagtgaaa ttctacaagt 4980

gtccctttta aaaatagtgt gtgctaacta agggctattc attctgccat ttttaacttg     5040

agacacattt aagaataaag ttggatgttc tttttctgtg attatgaaat gcaataaaat     5100

ctgaataaag ggcaaagttt cttcctgatc agattagatt aagtgcttag ttttattcct     5160

aattctgccc cttttaacct gatgtgtacc ttactctttc ctccctctct tcctcccttc     5220

ctcctctccc cactcctttc ctgcatttct ccctccctcc ttccctcgtc acatctctta     5280

tatgagcaca gtaattatga cctttttgtt ttggctacaa cttgattgaa acaagttcaa     5340

aatttaaaca agttcataca agtttttaaa aattctcaac tggtagcttg cttgctgtgc     5400

gtcttccaaa ctaaagcctg caagcgccac aattctaagt gccgccttcc attttttttc     5460

agtatgtttt cactggctta tgttttcaga tatgattttc ctttttcgca tatatggttt     5520

aataaatggg ggataatttt ttttttgtaa atccgtgctt gtgccaagtc tcatatttct     5580

ttgccatctc agaattatct ttttaccacc actgtttata aaatttcctt agagactttt     5640

tgaagggaaa atagagcaac agggaaaaat gaaagaaatg tcttggttac tgagctctaa     5700

atagacaggt ttgatggcac ttctcatgat acattttagt tattcttata aaagcaaaca     5760

ggcaaacatg agtgtaaatt aaagacaaaa agaaaactct ggttttatat ttgagaacac     5820

gtgaaaaatc atgggtcaac ataaaatctt gagaaccttc tactttctct gggaaagcat     5880

tatatagtgg tgcattagtt tagaaagtca gctatgattt tgcctatagt tctagttatt     5940

agctttgggg ttttcttgta ctttaagaca tacctgtaaa ttgaacctat ttgaattata     6000

ttccactgta tgtgtattat ggctcttttc ctattagagc aacttgtgtt tccctgataa     6060

tgtgtacatt ttttaggcat gtacttaata gttcacaatg ttctaaattt ggaaggactt     6120

aaaaaaaaaa cttgtttaaa tttccatctg ttttgtaata tctagctcta tatgtaaatg     6180

atgggtttgt tggtatttaa aatgaataca acttgaatgt aattaaagtg ctgttttttg     6240

gaagcgataa actttaaata tacttattaa aatgaaattc tattaaaaaa aaaaaaaaaa     6300

a                                                                   6301

<210>  3
<211>  25
<212>  DNA
<213>  Artificial sequence

<220>
<223>  CSBP probe 1

<400>  3
acgaagtgat gggtgggagc gagcc                                          25

<210>  4
<211>  25
<212>  DNA

<213>  Artificial sequence

<220>
<223>  CSBP probe 2

<400>  4
cccataactg gagcaactgt tcacc                                          25


<210>  5
<211>  20
<212>  DNA
<213>  Artificial sequence

<220>
<223>  CSBP forward primer

<400>  5
gacgggaaag gattggtcaa                                                20


<210>  6
<211>  20
<212>  DNA
<213>  Artificial sequence

<220>
<223>  CSBP reverse primer

<400>  6
aaaaagcacg aagtgatggg                                                20


<210>  7
<211>  32
<212>  DNA
<213>  Artificial sequence

<220>
<223>  rCSBP forward primer

<400>  7
ccgaattcat gtccaggccg agcagcgtct cc                                  32


<210>  8
<211>  33
<212>  DNA
<213>  Artificial sequence

<220>
<223>  rCSBP reverse primer

<400>  8
ccctcgagtc aatcagttgt gtttttttct ccc                                 33


<210>  9
<211>  21
<212>  DNA
<213>  Artificial sequence

<220>

<223>  siRNA #127336

<400>  9
ggacauucgc auugaugagt t                                                21


<210>  10
<211>  21
<212>  DNA
<213>  Artificial sequence

<220>
<223>  siRNA #127337

<400>  10
gggagcaaau agauaccuat t                                                21


<210>  11
<211>  21
<212>  DNA
<213>  Artificial sequence

<220>
<223>  siRNA #127338

<400>  11
ccuguaauuu gacucauaat t                                                21


<210>  12
<211>  21
<212>  DNA
<213>  Artificial sequence

<220>
<223>  siRNA #30311

<400>  12
ggagcuuuca gugaccauat t                                                21


<210>  13
<211>  21
<212>  DNA
<213>  Artificial sequence

<220>
<223>  siRNA #30403

<400>  13
ggucaaauaa uaguaggaat t                                                21


<210>  14
<211>  21
<212>  DNA
<213>  Artificial sequence

<220>
<223>  siRNA #30217

<400>  14

ggccucucaa aaacgugcat t                                                21

<210>  15
<211>  17
<212>  DNA
<213>  Artificial sequence

<220>
<223>  HCV forward primer

<400>  15
cgggagagcc atagtgg                                                     17

<210>  16
<211>  19
<212>  DNA
<213>  Artificial sequence

<220>
<223>  HCV reverse primer

<400>  16
agtaccacaa ggcctttcg                                                   19

<210>  17
<211>  21
<212>  DNA
<213>  Artificial sequence

<220>
<223>  HCV probe

<400>  17
ctgcggaacc ggtgagtaca c                                                21

<210>  18
<211>  20
<212>  DNA
<213>  Artificial sequence

<220>
<223>  CSBP forward primer

<400>  18
gtgtctggac cccatcctta                                                  20

<210>  19
<211>  20
<212>  DNA
<213>  Artificial sequence

<220>
<223>  CSBP reverse primer

<400>  19
cccatcactt cgtgcttttt                                                  20

<210>  20
<211>  19
<212>  DNA
<213>  Artificial sequence

<220>
<223>  G6PDH forward primer

<400>  20
ctgcgttatc ctcaccttc                                                    19


<210>  21
<211>  18
<212>  DNA
<213>  Artificial sequence

<220>
<223>  G6PDH reverse primer

<400>  21
cggacgtcat ctgagttg                                                     18


<210>  22
<211>  28
<212>  DNA
<213>  Artificial sequence

<220>
<223>  G6PDH 3' FITC labeled probe

<400>  22
ggggctattt cgatgaattt gggatcat                                          28


<210>  23
<211>  27
<212>  DNA
<213>  Artificial sequence

<220>
<223>  G6PDH 5' LCRed640 labeled probe

<400>  23
cgggacgtga tgcagaacca cctactg                                           27


<210>  24
<211>  20
<212>  DNA
<213>  Artificial sequence

<220>
<223>  CSBP -4292 forward primer

<400>  24
gtcccaggag acaactccat                                                   20


<210>  25
<211>  23
<212>  DNA

<213>  Artificial sequence

<220>
<223>  CSBP +2412 reverse primer

<400>  25
catcagatcc ttgacttggt agg                                                23


<210>  26
<211>  29
<212>  DNA
<213>  Artificial sequence

<220>
<223>  CSBP -927 forward primer

<400>  26
tttgagctca atattccgcc cccttctta                                          29


<210>  27
<211>  29
<212>  DNA
<213>  Artificial sequence

<220>
<223>  CSBP -44 reverse primer

<400>  27
tttaagcttg ctagctgaca gccaatgac                                          29


<210>  28
<211>  31
<212>  DNA
<213>  Artificial sequence

<220>
<223>  CSBP -464 reverse primer

<400>  28
tttaagctta ggttcctacc ttcatggagt t                                       31


<210>  29
<211>  29
<212>  DNA
<213>  Artificial sequence

<220>
<223>  CSBP -463 forward primer

<400>  29
tttgagctct ggctctctgg ttctccaat                                          29


<210>  30
<211>  420
<212>  DNA
<213>  Homo sapiens

<400>  30

tggctctctg gttctccaat gtacatcgaa ggcgtggcat aatgccaggt ccacagtaga 60

agcccataca tgcttactgc aggaattaat gaaggactaa acgaagtagc aagtgagtga 120

ataagaagat gaggagcgac ccttggtggg cggcgcggga agagaaccgc accatagctc 180

cgcagacagc ctcctttagg cgcgcagcgg cggccgcgct gaggtcacgg gcgccggccc 240

tggtgacgtc attacgcccg cgctcctcgc ctggccgtga tatcaatggc gcaggcttca 300

cttctgctgt ggcggtgact gaggcctttc tggtgacctc agcccaacac aggccgtctc 360

cggagcttcc cggtagtggc cccggattcc cacggtcttt gtcattggct gtcagctagc 420

<210> 31
<211> 30
<212> DNA
<213> Artificial sequence

<220>
<223> CSBP. m-CREB F

<400> 31
cgccggccct ggtgtggtca ttacgcccgc 30

<210> 32
<211> 30
<212> DNA
<213> Artificial sequence

<220>
<223> CSBP. m-CREB R

<400> 32
gcgggcgtaa tgaccacacc agggccggcg 30

<210> 33
<211> 30
<212> DNA
<213> Artificial sequence

<220>
<223> CSBP. m-GATA F

<400> 33
ctcgcctggc cgttgtatca atggcgcagg 30

<210> 34
<211> 30
<212> DNA
<213> Artificial sequence

<220>
<223> CSBP. m-GATA R

<400> 34
cctgcgccat tgatacaacg gccaggcgag 30

<210>  35
<211>  37
<212>  DNA
<213>  Artificial sequence

<220>
<223>  CSBP. m-AP-1 F

<400>  35
acttctgctg tggcggtagt tgaggccttt ctggtga                                37


<210>  36
<211>  37
<212>  DNA
<213>  Artificial sequence

<220>
<223>  CSBP. m-AP-1 R

<400>  36
tcaccagaaa ggcctcaact accgccacag cagaagt                                37


<210>  37
<211>  463
<212>  DNA
<213>  Homo sapiens

<400>  37
tggctctctg gttctccaat gtacatcgaa ggcgtggcat aatgccaggt ccacagtaga      60

agcccataca tgcttactgc aggaattaat gaaggactaa acgaagtagc aagtgagtga     120

ataagaagat gaggagcgac ccttggtggg cggcgcggga agagaaccgc accatagctc     180

cgcagacagc ctcctttagg cgcgcagcgg cggccgcgct gaggtcacgg gcgccggccc     240

tggtgacgtc attacgcccg cgctcctcgc ctggccgtga tatcaatggc gcaggcttca     300

cttctgctgt ggcggtgact gaggcctttc tggtgacctc agcccaacac aggccgtctc     360

cggagcttcc cggtagtggc cccggattcc cacggtcttt gtcattggct gtcagctagc     420

aggcctggcc gctcccgtgc ggagagacca tctcttcagg gca                       463


<210>  38
<211>  25
<212>  DNA
<213>  Artificial sequence

<220>
<223>  RASSF1A forward primer

<400>  38
cagattgcaa gttcacctgc cacta                                             25


<210>  39
<211>  26
<212>  DNA
<213>  Artificial sequence

<220>
<223>  RASSF1A reverse primer

<400>  39
gatgaagcct gtgtaagaac cgtcct                                          26